# EUROPEAN PATENT APPLICATION

(11) **EP 4 052 727 A1**
(43) Date of publication of application: **07.09.2022**
(21) Application number: 20880352.8
(22) Date of filing: 22.10.2020
(51) Int. Cl.: A61K 45/00, A61K 39/395, A61P 9/00, A61P 11/00, A61P 13/12, A61P 17/04, A61P 17/06, A61P 19/02, A61P 25/00, A61P 29/00, A61P 35/00, C12N 5/0783, C12P 21/02, C12Q 1/02

(54) **IMMUNE RESPONSE SUPPRESSOR**

(30) Priority: 30.10.2019 JP 2019197223
(71) Applicant: University of Tsukuba, Ibaraki 305-8577 (JP)
(72) Inventor: SHIBUYA Kazuko, Tsukuba-shi, Ibaraki 305-8577 (JP); SHIBUYA Akira, Tsukuba-shi, Ibaraki 305-8577 (JP); HAYASHI Kyoko, Tsukuba-shi, Ibaraki 305-8577 (JP)
(74) Representative: Dehns
(86) International application number: PCT/JP2020/039693
(87) International publication number: WO 2021/085295

(57) **Abstract**

An object is to elucidate the immune response mechanism of IL-17-producing cells which causes a pathological condition such as psoriasis, and to provide an immune response suppressant for suppressing the immune response of IL-17-producing cells, a medicament for treating or preventing a disease or a pathological condition involving the immune response of an IL-17-producing cell, a method for inducing immune response in γδT cells, and a method for evaluating a medicament (candidate substance) and a method for producing IL-17 by use of the method. The present invention provides an immune response suppressant for an IL-17-producing cell, including a substance that inhibits the binding of CD96 to at least one protein selected from CD155 and CD111, a medicament including the immune response suppressant for an IL-17-producing cell, a method having a step (A) of culturing at least one of a γδT cell and a CD4-positive T cell together with IL-23, an anti-CD3 antibody capable of stimulating a TCR/CD3 complex and an anti-CD96 antibody capable of stimulating CD96, and a method for evaluating a medicament (candidate substance) and a method for producing IL-17, including a method having the step (A).

SEQUENCE LISTING

<110>  University of Tsukuba

<120>  Suppressant of immune response

<130>  SF-3643

<160>  19

<170>  PatentIn version 3.5

<210>  1
<211>  21
<212>  DNA
<213>  Artificial sequence

<220>
<223>  Forward primer for IL-17

<400>  1
tttaactccc ttggcgcaaa a                                                21


<210>  2
<211>  20
<212>  DNA
<213>  Artificial sequence

<220>
<223>  Reverse primer for IL-17

<400>  2
ctttccctcc gcattgacac                                                  20


<210>  3
<211>  23
<212>  DNA
<213>  Mus musculus

<400>  3
aatagagaca aatcggactc tgg                                              23


<210>  4
<211>  21
<212>  DNA
<213>  Artificial sequence

<220>
<223>  Forward primer for IL-23

<400>  4
cagctctctc ggaatctctg c                                                21


<210>  5
<211>  20
<212>  DNA
<213>  Artificial sequence

<220>

<223>  Reverse primer for IL-23

<400>  5
gaccttggcg gatcctttgc                                                   20


<210>  6
<211>  20
<212>  DNA
<213>  Artificial sequence

<220>
<223>  Forward primer for IL-23R

<400>  6
gcaacatgac atgcacctgg                                                   20


<210>  7
<211>  20
<212>  DNA
<213>  Artificial sequence

<220>
<223>  Reverse primer for IL-23R

<400>  7
gacagcttgg acccatacca                                                   20


<210>  8
<211>  19
<212>  DNA
<213>  Artificial sequence

<220>
<223>  Forward primer for IL-12Rbeta1

<400>  8
atggctgctg cgttgagaa                                                    19


<210>  9
<211>  22
<212>  DNA
<213>  Artificial sequence

<220>
<223>  Reverse primer for IL-12Rbeta1

<400>  9
agcactcata gtctgtcttg ga                                                22


<210>  10
<211>  20
<212>  DNA
<213>  Artificial sequence

<220>
<223>  Forward primer for IL-22

<400>  10

tttcctgacc aaactcagca                                                    20

<210>  11
<211>  20
<212>  DNA
<213>  Artificial sequence

<220>
<223>  Reverse primer for IL-22

<400>  11
ctggatgttc tggtcgtcac                                                    20

<210>  12
<211>  110
<212>  PRT
<213>  Mus musculus

<220>
<221>  misc_feature
<223>  TX111.2 light chain variable region

<400>  12

Val Met Thr Gln Ser Pro Leu Ser Leu Pro Val Ser Leu Gly Asp Gln
1               5                   10                  15

Ala Ser Ile Ser Cys Arg Ser Ser Gln Ser Ile Ala His Ser Asn Gly
            20                  25                  30

Asn Thr Tyr Leu Glu Trp Tyr Leu Gln Lys Pro Gly Gln Ser Pro Lys
        35                  40                  45

Leu Leu Ile Tyr Lys Val Ser Asn Arg Phe Ser Gly Val Pro Asp Arg
    50                  55                  60

Phe Ser Gly Ser Gly Ser Gly Thr Asp Phe Thr Leu Lys Ile Ser Arg
65                  70                  75                  80

Val Glu Ala Glu Asp Leu Gly Val Tyr Tyr Cys Phe Gln Gly Ser His
                85                  90                  95

Val Pro Trp Thr Phe Gly Gly Gly Thr Lys Leu Glu Ile Lys
            100                 105                 110

<210>  13
<211>  117
<212>  PRT
<213>  Mus musculus

<220>
<221>  misc_feature

<223>  TX111.2 heavy chain variable region

<400>  13

Gln Val Gln Leu Gln Gln Pro Gly Ala Glu Leu Val Lys Pro Gly Ala
1               5                   10                  15

Ser Val Lys Leu Ser Cys Lys Thr Ser Gly Tyr Ile Phe Thr Arg Tyr
            20                  25                  30

Trp Met His Trp Val Lys Gln Arg Pro Gly Gln Gly Leu Glu Trp Ile
        35                  40                  45

Ala Leu Ile His Pro Asn Ser Gly Ser Thr Asn Tyr Asn Glu Lys Phe
    50                  55                  60

Lys Ser Lys Ala Thr Leu Thr Val Asp Lys Ser Ser Ser Thr Ala Tyr
65                  70                  75                  80

Met Gln Leu Ser Arg Leu Thr Ser Glu Asp Ser Ala Val Tyr Tyr Cys
                85                  90                  95

Val Asn Asp Gly Asn Gly Thr Met Asp Tyr Trp Gly Gln Gly Thr Ser
            100                 105                 110

Val Thr Val Ser Ser
        115

<210>  14
<211>  11
<212>  PRT
<213>  Mus musculus

<220>
<221>  misc_feature
<223>  CDR1 of TX111.2 light chain variable region

<400>  14

Gln Ser Ile Ala His Ser Asn Gly Asn Thr Tyr
1               5                   10

<210>  15
<211>  3
<212>  PRT
<213>  Mus musculus

<220>
<221>  misc_feature
<223>  CDR2 of TX111.2 light chain variable region

<400>  15

Lys Val Ser
1

<210> 16
<211> 9
<212> PRT
<213> Mus musculus

<220>
<221> misc_feature
<223> CDR3 of TX111.2 light chain variable region

<400> 16

Phe Gln Gly Ser His Val Pro Trp Thr
1               5

<210> 17
<211> 8
<212> PRT
<213> Mus musculus

<220>
<221> misc_feature
<223> CDR1 of TX111.2 heavy chain variable region

<400> 17

Gly Tyr Ile Phe Thr Arg Tyr Trp
1               5

<210> 18
<211> 8
<212> PRT
<213> Mus musculus

<220>
<221> misc_feature
<223> CDR2 of TX111.2 heavy chain variable region

<400> 18

Ile His Pro Asn Ser Gly Ser Thr
1               5

<210> 19
<211> 10
<212> PRT
<213> Mus musculus

<220>
<221> misc_feature
<223> CDR3 of TX111.2 heavy chain variable region

<400>  19

Val Asn Asp Gly Asn Gly Thr Met Asp Tyr
1               5                   10

## Description

### Technical Field

The present invention relates to an immune response suppressant.

### Background Art

Immunity includes natural immunity in which foreign substance that has invaded the body is recognized and immediately eliminated , and acquired immunity in which lymphocytes recognize information on foreign substance that has invaded the body, and particular foreign substance is eliminated on the basis of the information. The type of leading immunocytes differs depending on each immunity. For example, cells responsible for the natural immunity include, for example, macrophages, neutrophils, mast cells, and dendritic cells, and are characterized by recognizing a molecular structure common in pathogens or by being activated by cytokines and responding quickly to infection. On the other hand, cells responsible for the acquired immunity are T cells and B cells and are characterized by being activated when T cell receptors or B cell receptors recognize specific pathogens, and reacting strongly with infection, albeit responding slowly.

Most of T cells contained in human peripheral blood are αβT cells expressing a T cell receptor (TCR) composed of two glycoproteins called α and β chains, whereas several % of γδT cells exist which express TCR composed of γ and δ chains. The γδT cells, despite being one type of T cell, are regarded as cells positioned at the boundary between the natural immunity and the acquired immunity, for example, because they are instantly activated by cytokines to produce inflammatory cytokines. The γδT cells are localized with high frequency in tissues, such as intestinal, lung, genital intraepithelial, and skin tissues, which are exposed to extrinsic antigens. IL (interleukin)-17 and IFN (interferon)-y are known as inflammatory cytokines produced by the γδT cells.

Most of matured T cells contained in human peripheral blood express either CD4 or CD8 as a cell surface marker molecule. The T cells expressing CD4 function as helper T cells while the T cells expressing CD8 function as killer T cells. Two subsets called type 1 helper T cells (Th1 cells) and type 2 helper T cells (Th2 cells) had been known from long ago as the helper T cells. The Th1 cells are involved mainly in cell-mediated immunity or the elimination of intracellular parasites by producing IFN-γ and inducing macrophage activation. The Th2 cells are involved in humoral immunity or defense against infection with extracellular parasites, for example, by inducing the differentiation of eosinophils via IL-5 or IL-13 production. Then, IL-17-producing T cells have been discovered, designated as Th17 cells, and recognized as a subset different from the Th1 cells or the Th2 cells. The Th17 cells have been found to produce IL-17, GM-CSF (granulocyte-monocyte colony-stimulating factor), or IL-22 and to play a central role in, for example, autoimmunity, allergic response, and defense against infection with extracellularly growing bacteria.

ILC (innate lymphoid cell) cells are a recently identified lymphocyte group different from T cells or B cells, and reside in a wide range of tissues. They are classified into groups 1 to 3 depending on cytokines to be produced. The group 1 ILC (ILC1) is characterized by the production of a cytokine similar to that of Th1 cells, i.e., IFN-γ. The group 2 ILC (ILC2) is similar in cytokine production to Th2 cells and produces IL-5 and IL-13. The group 3 ILC (ILC3) is similar in cytokine production to Th17 cells and is found to produce IL-17 and IL-22.

IL-17 which is produced in the immune response of cells such as γδT cells, Th17 cells, or ILC3 cells is an inflammatory cytokine. While playing an important role in preventing fungal infection, IL-17 is known to induce inflammation by inducing various factors such as inflammatory cytokines, chemokines, and cell adhesion factors through the action on various cells such as fibroblasts, epithelial cells, vascular endothelial cells, and macrophages. Thus, autoimmune diseases or chronic inflammatory diseases may be caused unless the production of IL-17 is properly controlled. It is known that IL-17 is overproduced in autoimmune diseases such as psoriasis, rheumatoid arthritis, multiple sclerosis, scleroderma, or lupus. It has also been reported that cells producing IL-17 are involved in the deterioration of pathological conditions in, for example, collagen-induced arthritis models which are used as rheumatoid arthritis models, EAE (experimental autoimmune encephalomyelitis) models which are used as, for example, multiple sclerosis or acute disseminated encephalomyelitis models, atopic dermatitis models, and DMBA/TPA-induced skin tumor models (Non Patent Literature 1). Further, it has been reported that γδT cells producing IL-17 are also involved in the deterioration of pathological conditions in cerebral infarction models and nephritis models (Non Patent Literatures 2 and 3). Moreover, it has been reported that in lung injury and pulmonary fibrosis patients as well, the expression level of IL-17 is increased and IL-17 production by, for example, γδT cells, Th17 cells, or ILC3 cells is involved in pathological conditions (Non Patent Literature 4) .

Thus, IL-17 is targeted for treating or preventing these diseases. In actuality, IL-17 inhibitors containing an anti-IL-17A antibody formulation secukinumab or ixekizumab or an anti-IL-17A receptor antibody formulation brodalumab have been developed and used in psoriasis treatment (Non Patent Literature 5).

CD96 is a type I transmembrane glycoprotein belonging to the immunoglobin superfamily and is reportedly expressed in cells originating in the hematopoietic system, particularly, NK cells (natural killer cells), αβT cells, and γδT cells (Non Patent Literature 6) for example. CD96 is directed to CD155 or CD111 as a ligand expressed on target cells, and activated thereby. It is also known for CD96 that CD96 expressed in NK cells is involved in tumor immunity as an immune checkpoint receptor that transmits inhibitory signals (Non Patent Literature 7). However, much remains unknown about the role of CD96 in the immunosuppressive system.

### Citation List

### Non Patent Literature

Non Patent Literature 1: Monin et al., IL-17 family cytokines: signaling mechanisms, biological activities, and therapeutic implications, Cold Spring Hab Pespect Biol., 2018, 10 (4)
Non Patent Literature 2: Shichita et al., Pivotal role of cerebral interleukin-17-producing gamma-delta-T-cells in the delayed phase of ischemic brain injury. Nature Medicine, 2009, vol. 15, number 8, 946-951
Non Patent Literature 3: Turner et al., IL-17A Production by Renal Gamma-delta-T-cells Promotes Kidney injury in Crescentic GN. J Am Soc Nephrol, 2012, 23. 1486-1495
Non Patent Literature 4: Gurczynski et al., IL-17 in the lung: the good, the bad, and the ugly, Am J Physiol Lung Cell Mol Physiol, 2018, 314. L6-L16
Non Patent Literature 5: The Biologics Review Committee of the Japanese Dermatological Association for Psoriasis Formulated, Manual of the Japanese Dermatological Association, "Japanese guidance for use of biologics for psoriasis (the 2019 version)"
Non Patent Literature 6: Georgiev et al., Coming of Age: CD96 Emerges as Modulator of Immune Responses, frontiers in Immunology, 2018, Vol. 9, Article 1072
Non Patent Literature 7: Chan et al., The receptors CD96 and CD226 oppose each other in the regulation of natural killer cell functions, Nature Immunology, 2014, volume 15,431-438

### Summary of Invention

### Technical Problem

The present inventors have thought that if the production of IL-17 can be suppressed by elucidating the immune response mechanism of IL-17-producing cells and partially inhibiting the mechanism, this is effective for the treatment of pathological conditions involving cells producing IL-17. Accordingly, an object of the present invention is to elucidate the immune response mechanism of IL-17-producing cells which causes a pathological condition such as psoriasis, and to provide an immune response suppressant for suppressing the immune response of IL-17-producing cells and a medicament for treating or preventing a disease or a pathological condition involving the immune response of an IL-17-producing cell as the first and second aspects of the present invention. Another object thereof is to provide a method for inducing immune response in cells such as γδT cells as the third aspect of the present invention. A further object thereof is to provide a method for evaluating a medicament (candidate substance) for treating or preventing a disease or a pathological condition involving the immune response of an IL-17-producing cell as the fourth aspect of the present invention. An object thereof is to provide a method for producing IL-17 by inducing immune response in cells such as γδT cells as the fifth aspect of the present invention.

### Solution to Problem

The present inventors have conducted diligent studies to attain the objects. As a result, they have found that the objects can be attained by the following configurations, leading to the completion of the present invention.

The present invention relates to, for example, the following [1] to [11].
[1] An immune response suppressant for an IL-17-producing cell, comprising a substance that inhibits the binding of CD96 to at least one protein selected from CD155 and CD111.
[2] The immune response suppressant for an IL-17-producing cell according to [1], wherein the substance that inhibits the binding of CD96 to at least one protein selected from CD155 and CD111 is an anti-CD96 antibody.
[3] The immune response suppressant for an IL-17-producing cell according to [1] or [2], wherein the IL-17-producing cell is a γδT cell.
[4] The immune response suppressant for an IL-17-producing cell according to any of [1] to [3], wherein the immune response suppressant for an IL-17-producing cell is an IL-17 production suppressant or an IL-22 production suppressant.
[5] A medicament for treating or preventing a disease or a pathological condition involving the immune response of an IL-17-producing cell, comprising the immune response suppressant for an IL-17-producing cell according to any of [1] to [4] .
[6] The medicament according to [5], wherein the disease or the pathological condition involving the immune response of an IL-17-producing cell is psoriasis, rheumatoid arthritis, atopic dermatitis, multiple sclerosis, cerebral infarction, nephritis, lung injury, pulmonary fibrosis or skin tumor.
[7] A method comprising a step (A) of culturing at least one of a γδT cell and a CD4-positive T cell together with IL-23, an anti-CD3 antibody capable of stimulating a TCR/CD3 complex and an anti-CD96 antibody capable of stimulating CD96.
[8] The method according to [7], further comprising a step (B) of evaluating IL-17 after performing the step (A).
[9] A method for evaluating a medicament for treating or preventing a disease or a pathological condition involving the immune response of an IL-17-producing cell, comprising the method according to [8].
[10] The method for evaluating a medicament according to [9], wherein the disease or the pathological condition involving the immune response of an IL-17-producing cell is psoriasis, rheumatoid arthritis, atopic dermatitis, multiple sclerosis, cerebral infarction, nephritis, lung injury, pulmonary fibrosis or skin tumor.
[11] A method for producing IL-17, comprising a step (A) of culturing at least one of a γδT cell and a CD4-positive T cell together with IL-23, an anti-CD3 antibody capable of stimulating a TCR/CD3 complex and an anti-CD96 antibody capable of stimulating CD96.

### Advantageous Effects of Invention

The present invention can provide an immune response suppressant for suppressing the immune response of IL-17-producing cells which causes a pathological condition such as psoriasis, and a medicament for treating or preventing a disease or a pathological condition involving the immune response of an IL-17-producing cell. Also, a method for inducing immune response in γδT cells can be provided. Further, a method for evaluating a medicament for treating or preventing a disease or a pathological condition involving the immune response of an IL-17-producing cell can be provided. A method for producing IL-17 by inducing immune response in γδT cells can be provided.

### Brief Description of Drawings

[Figure 1] Figure 1 is photographs of the backs on day 3 from the start of imiquimod application of wild-type mice and CD96-deficient mice as imiquimod-induced psoriasis models.
[Figure 2] Figure 2 is graphs of the erythema, scaling, and skin thickness of the back skin on day 3 from the start of imiquimod application in imiquimod-induced psoriasis models. The ordinates depict an erythema score, a scaling score, and a skin thickness (µm), respectively.
[Figure 3] Figure 3 is photographs of HE-stained tissue preparations of the mouse back skin on day 3 from the start of imiquimod application in imiquimod-induced psoriasis models.
[Figure 4] Figure 4 is a graph showing the epidermal thickness of the mouse back skin on day 3 from the start of imiquimod application in imiquimod-induced psoriasis models.
[Figure 5] Figure 5 is a graph showing the number of neutrophils in the mouse back skin on day 3 from the start of imiquimod application in imiquimod-induced psoriasis models. The ordinate depicts the number of neutrophils per cm².
[Figure 6] Figure 6 is a graph showing the relative expression level of IL-23 in myeloid cells of the mouse back skin in imiquimod-induced psoriasis models.
[Figure 7] Figure 7 is graphs showing the relative expression levels of IL-23R and IL-12β1 in γδT cells of the mouse back skin in imiquimod-induced psoriasis models.
[Figure 8] Figure 8 is graphs showing the relative expression levels of IL-17 and IL-22 in γδT cells of the mouse back skin in imiquimod-induced psoriasis models.
[Figure 9] Figure 9 is graphs showing the ratio of IL-17-producing cells and the number of IL-17-producing cells in the mouse back skin in imiquimod-induced psoriasis models.
[Figure 10] Figure 10 is a graph showing difference in relative IL-17 expression level increased by IL-23 in wild-type mouse spleen-derived γδT cells in the presence or absence of stimulation with an immobilized CD96 antibody.
[Figure 11] Figure 11 is a graph showing difference in relative IL-17 expression level increased by CD3 signals and IL-23 in wild-type mouse spleen-derived γδT cells in the presence or absence of stimulation with an immobilized anti-CD96 antibody.
[Figure 12] Figure 12 is graphs showing difference in the activation by CD3 signals and IL-23 of wild-type mouse spleen-derived γδT cells in the presence or absence of stimulation with an immobilized anti-CD96 antibody. The ordinates each depict a mean of fluorescence.
[Figure 13] Figure 13 shows results of flow cytometry analysis to confirm the expression of CD96 in human PBMC-derived γδT cells and human PBMC-derived CD4+ T cells. The abscissa depicts the expression intensity of CD96, and the ordinate depicts the number of cells.
[Figure 14] Figure 14 is a graph showing difference in the amount of IL-17 produced increased by CD3 signals and IL-23 in human PBMC-derived γδT cells obtained from 7 healthy persons in the presence or absence of stimulation with an immobilized anti-CD96 antibody.
[Figure 15] Figure 15 is graphs showing difference in the activation by CD3 signals of human PBMC-derived γδT cells obtained from 6 healthy persons in the presence or absence of stimulation with an immobilized anti-CD96 antibody. The ordinates each depict a mean of fluorescence.
[Figure 16] Figure 16 is a graph showing difference in the amount of IL-17 produced increased by CD3 signals and IL-23 in human PBMC-derived CD4+ T cells obtained from 8 healthy persons in the presence or absence of stimulation with an immobilized anti-CD96 antibody.
[Figure 17] Figure 17 is graphs showing difference in the activation by CD3 signals of human PBMC-derived CD4+ T cells obtained from 6 healthy persons in the presence or absence of stimulation with an immobilized anti-CD96 antibody. The ordinates each depict a mean of fluorescence.
[Figure 18] Figure 18 is a graph showing difference in IL-17 production elevated by CD3 signals and simulation with IL-23 and CD155 in human PBMC-derived CD4+ T cells obtained from healthy persons in the presence or absence of an anti-CD96 neutralizing antibody.
[Figure 19] Figure 19 shows results of flow cytometry analysis to confirm the binding specificity of an anti-mouse CD96 neutralizing antibody (TX111.2) for CD96. The abscissa depicts the expression intensity of CD96, and the ordinate depicts the number of cells.
[Figure 20] Figure 20 is a graph showing that the anti-mouse CD96 neutralizing antibody (TX111.2) inhibits the binding of CD96 to CD155 in a concentration-dependent manner. The ordinate depicts a mean of fluorescence.
[Figure 21] Figure 21 is a diagram showing the amino acid sequences of the heavy chain and light chain variable regions of the anti-mouse CD96 neutralizing antibody (TX111.2).

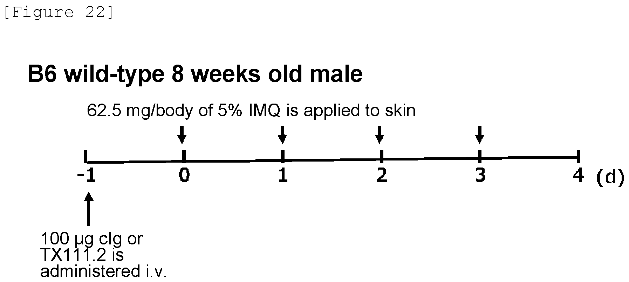

[Figure 22] Figure 22 is a conceptual diagram showing the protocol of a prophylactic administration experiment of the anti-mouse CD96 neutralizing antibody (TX111.2) to imiquimod-induced psoriasis mouse models.
[Figure 23] Figure 23 is photographs of the backs on day 3 from the start of imiquimod application of a control antibody (cIg) administration group and a TX111.2 administration group in a prophylactic administration experiment of the anti-mouse CD96 neutralizing antibody (TX111.2) to imiquimod-induced psoriasis mouse models.
[Figure 24] Figure 24 is graphs of the erythema, scaling, and skin thickness of the back skin on day 4 from the start of imiquimod application in a prophylactic administration experiment of the anti-mouse CD96 neutralizing antibody (TX111.2) to imiquimod-induced psoriasis mouse models. The ordinates depict an erythema score, a scaling score, and a skin thickness (µm), respectively.
[Figure 25] Figure 25 is images of HE-stained skin tissues (left) and a graph of an epidermal thickness (right) of the back on day 4 from the start of imiquimod application in a prophylactic administration experiment of the anti-mouse CD96 neutralizing antibody (TX111.2) to imiquimod-induced psoriasis mouse models.

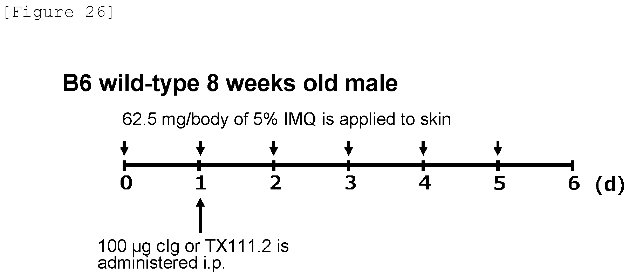

[Figure 26] Figure 26 is a conceptual diagram showing the protocol of a therapeutic administration experiment of the anti-mouse CD96 neutralizing antibody (TX111.2) to imiquimod-induced psoriasis mouse models.
[Figure 27] Figure 27 is photographs of the backs on day 6 from the start of imiquimod application of a control antibody (cIg) administration group and a TX111.2 administration group in a therapeutic administration experiment of the anti-mouse CD96 neutralizing antibody (TX111.2) to imiquimod-induced psoriasis mouse models.
[Figure 28] Figure 28 is graphs of the erythema, scaling, and skin thickness of the back skin on day 1 (D1) and day 6 (D6) from the start of imiquimod application in a therapeutic administration experiment of the anti-mouse CD96 neutralizing antibody (TX111.2) to imiquimod-induced psoriasis mouse models. The ordinates depict an erythema score, a scaling score, and a skin thickness (µm), respectively.
[Figure 29] Figure 29 is images of HE-stained skin tissues (left) and a graph of an epidermal thickness (right) of the back on day 6 from the start of imiquimod application in a therapeutic administration experiment of the anti-mouse CD96 neutralizing antibody (TX111.2) to imiquimod-induced psoriasis mouse models.
[Figure 30] Figure 30 is a conceptual diagram showing the protocol of an administration experiment of the anti-mouse CD96 neutralizing antibody (TX111.2) to EAE mouse models.
[Figure 31] Figure 31 is a graph showing time-dependent change in EAE pathological condition score in an administration experiment of the anti-mouse CD96 neutralizing antibody (TX111.2) to EAE mouse models.

### Description of Embodiments

The present invention broadly includes five aspects.

The first aspect is an immune response suppressant for an IL-17-producing cell, comprising a substance that inhibits the binding of CD96 to at least one protein selected from CD155 and CD111.

The second aspect is a medicament for treating or preventing a disease or a pathological condition involving the immune response of an IL-17-producing cell, comprising the immune response suppressant for an IL-17-producing cell of the first aspect.

The third aspect is a method comprising a step (A) of culturing at least one of a γδT cell and a CD4-positive T cell together with IL-23, an anti-CD3 antibody capable of stimulating a TCR/CD3 complex and an anti-CD96 antibody capable of stimulating CD96.

The fourth aspect is a method for evaluating a medicament (candidate substance) for treating or preventing a disease or a pathological condition involving the immune response of an IL-17-producing cell, comprising the method of the third aspect.

The fifth aspect is a method for producing IL-17, comprising a step (A) of culturing at least one of a γδT cell and a CD4-positive T cell together with IL-23, an anti-CD3 antibody capable of stimulating a TCR/CD3 complex and an anti-CD96 antibody capable of stimulating CD96.

Next, the present invention will be specifically described.

### <Substance that inhibits binding of CD96 to at least one protein selected from CD155 and CD111>

As mentioned later in Examples, the present inventors have revealed that the IL-17 production of γδT cells is increased through the activation of the γδT cells by CD96. Thus, if CD96 signals are inhibited, it is expected that the activation of γδT cells is suppressed so that the IL-17 production of the γδT cells is suppressed. Specifically, a substance that inhibits the binding of CD96 to at least one protein selected from CD155 and CD111 can be used as an immune response suppressant for an IL-17-producing cell.

One substance that inhibits the binding of CD96 to at least one protein selected from CD155 and CD111 may be used alone, or two or more thereof may be used in combination.

In the present invention, CD96, CD155 and CD111 each include those derived from mammals such as mice, rats, hamsters, guinea pigs, dogs, pigs, and primates including monkeys and humans. In the present invention, CD96, CD155 and CD111 are preferably human CD96, CD155 and CD111.

Examples of the substance that inhibits the binding of CD96 to at least one protein selected from CD155 and CD111 include specific binding substances for CD96, CD96 expression inhibitors, specific binding substances for CD155, CD155 expression inhibitors, specific binding substances for CD111, and CD111 expression inhibitors. A specific binding substance for CD96, a specific binding substance for CD155, or a specific binding substance for CD111 is preferred, and a specific binding substance for CD96 is more preferred, because the immune response of IL-17-producing cells can be efficiently suppressed.

The specific binding substance for CD96, the specific binding substance for CD155, or the specific binding substance for CD111 is not particularly limited as long as the substance can inhibit the binding of CD96 to at least one protein selected from CD155 and CD111. Examples thereof include antibodies, antibody fragments and aptamers directed to CD96 (anti-CD96 antibodies, anti-CD96 antibody fragments, and aptamers against CD96), antibodies, antibody fragments and aptamers directed to CD155 (anti-CD155 antibodies, anti-CD155 antibody fragments, and aptamers against CD155), and antibodies, antibody fragments and aptamers directed to CD111 (anti-CD111 antibodies, anti-CD111 antibody fragments, and aptamers against CD111). The antibodies may be prepared by immunizing animals such as mice or may be prepared by screening antibody libraries such as phage libraries. The antibody fragments can be antibody fragments containing antibody binding sites of the antibodies. Examples thereof include F(ab')₂, Fab', Fab, Fv, and scFv. Examples of the aptamers include nucleic acid aptamers and peptide aptamers. The specific binding substance for CD96, the specific binding substance for CD155, or the specific binding substance for CD111 is preferably an anti-CD96 antibody, an anti-CD155 antibody or an anti-CD111 antibody or a fragment thereof, more preferably an anti-CD96 antibody or a fragment thereof, because the immune response of IL-17-producing cells can be efficiently suppressed.

The specific binding substance for CD96 may be solubilized CD155 or solubilized CD111. Examples of the solubilized CD155 include fusion proteins of CD155 and antibody constant regions. Examples of the solubilized CD111 include fusion proteins of CD111 and antibody constant regions. The specific binding substance for CD155 or the specific binding substance for CD111 may be solubilized CD96. Examples of the solubilized CD96 include fusion proteins of CD96 and antibody constant regions.

The CD96 expression inhibitor, the CD155 expression inhibitor, or the CD111 expression inhibitor is not particularly limited as long as the substance reduces the expression of CD96, CD155, or CD111 and can consequently inhibit the binding of CD96 to CD155 or CD111. Examples thereof include siRNA, shRNA, miRNA, ribozymes, antisense nucleic acids, and low-molecular compounds. The siRNA, the shRNA, the miRNA, the ribozymes and the antisense nucleic acids may have various chemical modifications in order to improve stability or activity. For example, a phosphate residue may be replaced with, for example, a chemically modified phosphate residue such as phosphorothioate, methyl phosphonate, or phosphorodithioate in order to prevent degradation by hydrolase such as nuclease. Alternatively, at least a portion thereof may be composed of a nucleic acid analog such as a peptide nucleic acid (PNA).

### <Anti-CD96-related molecule antibody>

The anti-CD96 antibody, the anti-CD155 antibody and the anti-CD111 antibody (hereinafter, also referred to as an anti-CD96-related molecule antibody) can be prepared in accordance with an antibody or antiserum production method known in the art using the full-length proteins or partial proteins of CD96, CD155, and CD111 (hereinafter, also referred to as a CD96-related molecule) as antigens. The anti-CD96-related molecule antibody desirably binds to an expression site on cell surface. Therefore, the partial protein is desirably an extracellular region of the CD96-related molecule. These antigens can be prepared by protein expression and purification methods known in the art.

The anti-CD96-related molecule antibody may be any of a polyclonal antibody and a monoclonal antibody. A monoclonal antibody is preferred because titer is easily kept constant. The anti-CD96-related molecule antibody may be any of a mouse antibody, a rat antibody, a guinea pig antibody, a hamster antibody, a rabbit antibody, a monkey antibody, a dog antibody, a chimeric antibody, a humanized antibody, and a human antibody. The anti-CD96-related molecule antibody may be chemically modified in order to improve physical properties such as retention in blood.

The anti-CD96-related molecule antibody used may have an engineered constant region (Fc region). The constant region (Fc region) generally exerts an effector function such as ADCC (antibody-dependent cellular cytotoxicity) or complement-dependent cytotoxic activity (CDC activity) through interaction with an Fc receptor or a complement component. The effector function is known to be largely influenced by, for example, the structure of a composite sugar chain present in the antibody Fc region or the gene polymorphism of the Fc receptor. The anti-CD96-related molecule antibody is preferably engineered so as not to exert an effector function by introducing a mutation to the Fc region. The Fc region can be engineered in accordance with a method known in the art.

The isotype of the anti-CD96-related molecule antibody is not particularly limited and may be, for example, any of IgG, IgM, IgA, IgD, and IgE.

One anti-CD96-related molecule antibody may be used alone, or two or more thereof may be used in combination.

Examples of the antigen suitable for the preparation of the anti-CD96-related molecule antibody include cells forced to express the CD96-related molecule using expression vectors, and CD96-related molecule recombinant proteins prepared using CD96-related molecule expression plasmid vectors or CD96-related molecule expression virus vectors (e.g., adenovirus vectors). The CD96-related molecule recombinant proteins may be fused with other proteins or may be tagged, for example. The CD96-related molecule recombinant proteins are preferably chimeric proteins through fusion with, for example, IgG Fc, from the viewpoint of protein stability. One antigen for use in the preparation of the anti-CD96-related molecule antibody may be used alone, or two or more thereof may be used in combination. In the case of preparing, for example, an anti-CD96 antibody, cells forced to express CD96 are preferably used in combination with a CD96 recombinant protein because an antibody having high specificity can be efficiently prepared.

The polyclonal antibody can be prepared by a method known in the art. It can be prepared, for example, by immunizing an appropriate animal with an antigenic protein or a mixture of an antigenic protein and a carrier protein, collecting an antibody-containing material against the antigenic protein from the immunized animal, and separating or purifying the antibody, if necessary. Examples of the animal used generally include mice, rats, sheep, goats, rabbits, and guinea pigs. A complete Freund's adjuvant or an incomplete Freund's adjuvant can be administered together with the antigenic protein in order to enhance the ability to produce antibodies. The administration is generally performed a total of approximately 3 to 10 times (usually approximately once every two weeks). The polyclonal antibody can be collected from, for example, the blood or ascitic fluid of the animal immunized by the method described above. Polyclonal antibody titer in antiserum can be measured by ELISA. The polyclonal antibody can be separated or purified in accordance with, for example, an immunoglobulin separation or purification method such as a purification method using an antigen-bound solid phase or an active adsorbent such as protein A or protein G, a salting-out method, an alcohol precipitation method, an isoelectric precipitation method, electrophoresis, an adsorption-desorption method using an ion exchanger, an ultracentrifugation method, or a gel filtration method.

The monoclonal antibody can be prepared by a method known in the art. Specifically, a mammal, preferably a mouse, a rat, a hamster, a guinea pig or a rabbit, is subjected to immune sensitization by the subcutaneous, intramuscular, intravenous, intra-footpad, or intraperitoneal injection of the antigen once to several times, if necessary, together with a Freund's adjuvant. Usually, immunization is performed two to five times approximately every month from the initial immunization, and antibody-producing cells can be obtained from the immune-sensitized mammal approximately 3 or 4 days after final immunization. The number of shots and time intervals for immunization can be appropriately changed depending on, for example, the properties of the immunogen used.

Hybridomas that secrete the monoclonal antibody can be prepared in accordance with the method of Kohler and Milstein (Nature, 1975, vol. 256, p. 495-497) and a method equivalent thereto. Specifically, the hybridomas can be prepared by the cell fusion between antibody-producing cells contained in, for example, the spleen, lymph node, bone marrow or amygdala, preferably the spleen, obtained from the mammal immune-sensitized as mentioned above, and myeloma cells, which lack the ability to produce autoantibodies, derived from preferably a mammal such as a mouse, a rat, a guinea pig, a hamster, a rabbit or a human, more preferably a mouse, a rat or a human.

An established cell line, for example, P3-U1, NS-1, SP2, 653, X63, or AP-1, obtained from a mouse can be used as the myeloma cells for use in cell fusion.

A hybridoma clone producing the monoclonal antibody is screened for by culturing the hybridomas, for example, in a microtiter plate, measuring the reactivity of a culture supernatant in a well found to have growth with the antigen used in the mouse immune sensitization mentioned above by a measurement method such as RIA, ELISA, or FACS, and selecting a clone producing the monoclonal antibody that exhibits specific binding to the antigen or hapten. A method of immobilizing the antigen, and detecting the antibody in the culture supernatant that binds thereto using a secondary antibody labeled with, for example, a radioactive material, a fluorescent material, or an enzyme is usually used. In the case of using antigen-expressing cells, the hybridoma culture supernatant is added to the cells and then reacted with a fluorescently labeled secondary antibody. Then, the fluorescence intensity of the cells can be measured in a fluorescence detection apparatus such as a flow cytometer to detect a monoclonal antibody that can bind to the antigen of the present invention on the cell membranes.

The monoclonal antibody can be produced from the selected hybridoma clone by culturing the hybridoma *in vitro* or by culturing the hybridoma in, for example, the ascitic fluid of a mouse, a rat, a guinea pig, a hamster or a rabbit, preferably a mouse or a rat, more preferably a mouse, followed by isolation from the obtained culture supernatant or the ascitic fluid of the mammal. The *in vitro* culture can be carried out using a nutrient medium known in the art that is used for proliferating, maintaining and preserving hybridomas and for producing monoclonal antibodies into culture supernatants or every nutrient medium induced and prepared from a basal medium known in the art, according to various conditions such as the characteristics of the cell species to be cultured, the object of a test or research and a culture method.

The monoclonal antibody can be isolated or purified by subjecting the culture supernatant or the ascitic fluid mentioned above to saturated ammonium sulfate, ion-exchange chromatography (e.g., DEAE or DE52), or affinity chromatography using, for example, an anti-immunoglobulin column or a protein A column.

A recombinant antibody produced by use of a gene recombination technique by cloning an antibody gene from antibody-producing cells, for example, hybridomas, and integrating it into an appropriate vector, which is then transferred to a host may be used as the anti-CD96-related molecule antibody.

Specifically, mRNA encoding an antibody variable region is isolated from cells immortalized with, for example, an oncogene, from hybridomas producing the antibody of interest or immunocytes, for example, sensitized lymphocytes, producing the antibody. For the mRNA isolation, total RNA is prepared by a method known in the art, for example, a guanidine ultracentrifugation method, and mRNA is prepared using, for example, mRNA Purification Kit (manufactured by Pharmacia Biotech Inc.). cDNA of the antibody variable region is synthesized from the obtained mRNA using reverse transcriptase. The cDNA synthesis can be performed using, for example, AMV Reverse Transcriptase First-strand cDNA Synthesis Kit. 5'-RACE using 5'-Ampli FINDER RACE Kit (manufactured by Clontech Laboratories, Inc.) and PCR can be used in cDNA synthesis and amplification. The DNA fragment of interest is purified from the obtained PCR product and ligated with vector DNA. A recombinant vector is further prepared therefrom and transferred to, for example, *E. coli,* and the desired recombinant vector is prepared by selecting a colony. The nucleotide sequence of the DNA of interest is confirmed by a method known in the art, for example, a deoxy method.

Provided that DNA encoding the variable region of the antibody of interest is obtained, this is linked to DNA encoding the desired antibody constant region and the resultant can be integrated into an expression vector. Alternatively, the DNA encoding the antibody variable region may be integrated into an expression vector containing DNA of the antibody constant region. For the production of the antibody of interest, the antibody gene is integrated into an expression vector such that its expression is attained under the control of an expression control region, for example, an enhancer/promoter. Next, host cells can be transformed with this expression vector and allowed to express the antibody.

For the expression of the antibody gene, DNAs encoding the heavy chain and the light chain of the antibody may be integrated into separate expression vectors, with which a host can be cotransformed, or DNAs encoding the heavy chain and the light chain may be integrated into a single expression vector, with which a host can be transformed.

A so-called phage display method (Nature Biotechnology 23, 1105 (2005)) may be used as an antibody preparation method. Specifically, an antibody gene library prepared by a method known in the art using human or animal (e.g., rabbit, mouse, rat, or hamster) B lymphocytes as materials, or an antibody gene library completely synthesized by selection and engineering from human or animal germ line sequences is displayed on, for example, bacteriophages, *E. coli,* yeasts, the surface of cells such as animal cells, or ribosomes. In this respect, examples of the form of the antibody to be displayed on cell surface include IgG molecules, IgM molecules, Fab fragments, and single-chain Fv (scFv) fragments.

The antibody fragment gene thus obtained can be recombined with the corresponding region of an IgG antibody gene by a method known in the art to obtain an antibody gene. The gene thus obtained is integrated into an appropriate vector, which is then transferred to a host. The antibody can be produced by use of a gene recombination technique.

### <Specific binding substance for CD96>

CD96 is a transmembrane protein including two isoforms in humans. Isoform 1 is detected in acute myeloid leukemia and contains an additional amino acid as compared with isoform 2. For humans, isoform 2 is a more general form, and the putative domain structure of isoform 2 has three external immunoglobulin-like domains (domains 1, 2, and 3). Mouse CD96 exists as a single isoform and has three external immunoglobulin-like domains (domains 1, 2, and 3).

The specific binding substance for CD96 preferably binds to at least one of the CD96 isoforms. The specific binding substance for human CD96 preferably binds to CD96 isoform 2.

The specific binding substance for CD96 preferably binds to the amino acid sequence of at least one external immunoglobulin-like domain of CD96. The specific binding substance for CD96 may bind to the amino acid sequence of domain 1; domain 2; domain 3; domain 1 and domain 2; domain 1 and domain 3; domain 2 and domain 3; or domain 1, domain 2, and domain 3, and preferably binds to domain 1. The specific binding substance for CD96 may bind to other CD96 domains or amino acid sequences in addition to at least one extracellular immunoglobulin-like domain.

The specific binding substance for CD96 inhibits, blocks, or competes against the binding of CD96 to CD155. The specific binding substance for CD96 preferably inhibits, or blocks against the binding of CD96 to CD155 at least partially by binding to an extracellular domain of CD96 capable of interacting with CD155, or a portion thereof.

The specific binding substance for CD96 preferably inhibits or reduces CD96 signal transduction. The inhibition or reduction of CD96 signal transduction may be ascribable to the inhibition of, blocking of, or competition against the binding to CD155, or may be ascribable to the blocking of CD96-induced signal transduction that may usually occur in response to the binding to CD155. As an example, CD96 contains an immunoreceptor tyrosine-based inhibitory motif (ITIM). The specific binding substance for CD96 may have the ability to inhibit or reduce CD96 signal transduction mediated by ITIM.

### <Anti-CD96 antibody>

The anti-CD96 antibody is not particularly limited as long as the antibody has specificity for CD96. A commercially available anti-CD96 antibody or an anti-CD96 antibody known in the art may be used, TX111.2 disclosed in the present specification may be used, or it may be newly prepared by various methods known in the art. TX111.2 is preferred.

For example, a mouse anti-human CD96 monoclonal antibody manufactured by BioLegend, Inc. (clone: NK92.39, NO. 338402), a mouse anti-human CD96 monoclonal antibody manufactured by Santa Cruz Biotechnology, Inc. (clone: NK92.50.1, NO. sc-53574 and clone: NK92.39.1, NO. sc-53575), or a sheep anti-human CD96 polyclonal antibody manufactured by RSD (NO. AF6199) can be used as the commercially available anti-CD96 antibody. Further, four commercially available anti-human CD96 antibodies (1C8, NK92.39, 3H8, and MAA6359) described in Japanese Patent No. 6618908 can be used as the commercially available anti-CD96 antibody.

Other anti-CD96 antibodies known in the art can be obtained by, for example, the following methods described in Japanese Patent No. 6618908. Four CD96-knockout mice are immunized with an external domain protein of mouse CD96. Likewise, four CD96-knockout mice are immunized with a purified external domain protein of human CD96. The immunization procedure is performed approximately three times at 4-week intervals. The mice are allowed to bleed 10 to 12 days after the third immunization, and serum is titrated through antigen screening by ELISA. A mouse having the highest antibody titer is used in fusion. Alternatively, if the mice do not sufficiently respond, a further immunization procedure is attempted. Selected hybridomas are cloned, and mAb (monoclonal antibody) is purified from each clone. Then, each individual human or mouse mAb is screened for by use of screening assay for identifying anti-CD96 antibodies (binding assay of CD96 to CD155, ADCC assay, and assay on human leucocyte effector function regulation by human CD96 antibodies), or assay on mouse NK cell function regulation by anti-CD96 antibodies. The clone is optionally isotyped in order to identify antibodies, such as IgG2 and IgG4 antibodies, which are less likely to or fail to induce ADCC.

Further examples of other anti-CD96 antibodies known in the art include anti-human CD96 mouse monoclonal antibodies m1718, m1719, m1720, m1721, and m1722 described in WO2019/091449, and chimeric antibodies and humanized antibodies obtained therefrom.

For example, a mutant, a chimeric antibody, or a humanized antibody of a commercially available anti-CD96 antibody or an anti-CD96 antibody known in the art may be used as the anti-CD96 antibody.

The anti-CD96 antibody is preferably an antibody that inhibits or reduces CD96 signal transduction, i.e., an anti-CD96 neutralizing antibody, among antibodies having specificity for CD96.

Preferred examples of the anti-CD96 neutralizing antibody can include, but are not particularly limited to, a mouse anti-human CD96 monoclonal antibody manufactured by BioLegend, Inc. (clone: NK92.39, NO. 338402) and TX111.2 disclosed in the present specification.

The anti-CD96 neutralizing antibody is preferably an antibody having a light chain variable region comprising the following amino acid sequences:
the amino acid sequence of SEQ ID NO: 14 as CDR1, the amino acid sequence of SEQ ID NO: 15 as CDR2, and the amino acid sequence of SEQ ID NO: 16 as CDR3, and
a heavy chain variable region comprising the following amino acid sequences:
   the amino acid sequence of SEQ ID NO: 17 as CDR1, the amino acid sequence of SEQ ID NO: 18 as CDR2, and the amino acid sequence of SEQ ID NO: 19 as CDR3.

The anti-CD96 neutralizing antibody is preferably an antibody having a light chain variable region having the amino acid sequence of SEQ ID NO: 12 and a heavy chain variable region having the amino acid sequence of SEQ ID NO: 13.

The anti-CD96 neutralizing antibody is preferably an antibody having a light chain variable region having an amino acid sequence at least 90% or more identical to SEQ ID NO: 12 and a heavy chain variable region having an amino acid sequence at least 90% or more identical to SEQ ID NO: 13.

The anti-CD96 neutralizing antibody is preferably an antibody having a light chain variable region having an amino acid sequence that is derived from the amino acid sequence of SEQ ID NO: 12 by the substitution, addition, or deletion of one or several amino acids in a region other than CDR1 to CDR3, and is at least 90% or more identical to SEQ ID NO: 12, and a light chain variable region having an amino acid sequence that is derived from the amino acid sequence of SEQ ID NO: 13 by the substitution, addition, or deletion of one or several amino acids in a region other than CDR1 to CDR3, and is at least 90% or more identical to SEQ ID NO: 13.

### <IL-17>

IL-17 (interleukin-17) means the IL-17 family and encompasses IL-17A, IL-17B, IL-17C, IL-17D, IL-17E, and IL-17F. In the case of evaluating IL-17, at least one member of the IL-17 family can be evaluated. The IL-17 is preferably IL-17A because of a large amount of production and easy measurement.

### <IL-17-producing cell>

The IL-17-producing cell is not particularly limited as long as the cell produces IL-17. It is, for example, a γδT cell, a Th17 cell, or an ILC3 cell.

The γδT cell is a cell having a T cell receptor consisting of γ and δ chains on cell surface, and produces IL-17.

The Th17 cell (type 17 helper T cell) is a subset of helper T cells (Th cells) and is a cell that produces IL-17. Specifically, the Th17 cell can be regarded as a CD4+ T cell (CD4-positive T cell) producing IL-17.

The ILC3 cell requires a transcriptional factor RORγt and is a group 3 innate lymphoid cell (ILC) producing IL-17. The ILC3 cell includes ILC22 cells producing a lymphoid tissue inducer (LTi) and IL-22.

The IL-17-producing cell, particularly, the γδT cell, the Th17 cell, or the ILC3 cell, is also capable of producing IL-22.

The IL-17-producing cell is preferably a γδT cell because of large involvement in a disease or a pathological condition.

### <Immune response suppressant for IL-17-producing cell>

The immune response of IL-17-producing cells means a reaction that occurs in IL-17-producing cells among reactions that are performed in order to eliminate exogenous or endogenous foreign substance in specific response thereto, and may be ascribable to natural immunity or acquired immunity. The immune response of IL-17-producing cells is, for example, cytokine production. Examples thereof include the production of IL-17, IL-22, TNF, and IFN-γ.

The immune response of IL-17-producing cells may be immune reaction induced through a reaction that mimics a reaction that is performed in order to eliminate exogenous or endogenous foreign substance in specific response thereto. For example, when cells that have recognized foreign substance are known to produce a cytokine, the immune response of IL-17-producing cells induced by the addition of the cytokine in an *in vitro* test system is also included in the immune response of IL-17-producing cells. A method for inducing the immune response of IL-17-producing cells is not particularly limited and is preferably induction using a cytokine, more preferably induction using IL-23.

The immune response suppressant for an IL-17-producing cell may suppress cytokine production and can have action of suppressing any of various immune responses in IL-17-producing cells that are provoked by produced cytokines.

The immune response suppressant for an IL-17-producing cell is preferably a cytokine production suppressant, more preferably an IL-17 production suppressant or an IL-22 production suppressant.

The application of the immune response suppressant for an IL-17-producing cell is not particularly limited and is preferably use for treating or preventing a disease or a pathological condition involving the immune response of an IL-17-producing cell. The disease or the pathological condition involving the immune response of an IL-17-producing cell is not particularly limited as long as the disease or the pathological condition involves the immune response of an IL-17-producing cell. Psoriasis, rheumatoid arthritis, atopic dermatitis, multiple sclerosis, cerebral infarction, nephritis, lung injury, pulmonary fibrosis or skin tumor is preferred, psoriasis or multiple sclerosis is more preferred, and psoriasis is further preferred.

The treatment or prevention of the disease or the pathological condition includes, for example, the suppression of development of the disease or the pathological condition, the suppression of progression of the disease or the pathological condition, and the reduction or alleviation of symptoms of the disease or the pathological condition.

The psoriasis includes, for example, psoriasis vulgaris, psoriasis arthropica, psoriasis pustulosa, psoriasis guttata, and psoriatic erythroderma. The psoriasis is usually found to have erythema keratodes accompanied by thick silver gray scaling and can be pathologically diagnosed from the foundation of epidermal hyperplasia with the elongation of psoriasis-like rete ridges, parakeratosis with the absence of granular layers, and subcorneal neutrophilic microabscess.

The multiple sclerosis includes a relapsing-remitting form, primary progressive MS (PPMS), secondary progressive MS (SPMS), and progressing relapsing MS (PRMS). The multiple sclerosis can be diagnosed when a patient develops a single demyelinating symptom called CIS (clinically isolated syndrome) and the demyelinating symptom manifests temporal or spatial multiplicity. The immune response suppressant for an IL-17-producing cell of the present invention can be effective for use in the prevention or suppression of progression of CIS into multiple sclerosis.

The dose (effective amount) of the immune response suppressant for an IL-17-producing cell is not particularly limited and can be appropriately selected depending on, for example, the type or severity of symptoms of the disease or the pathological condition involving the immune response of an IL-17-producing cell, a recipient (e.g., age, sex, and body weight), and an administration route. When the substance that inhibits the binding of CD96 to at least one protein selected from CD155 and CD111 is, for example, an anti-CD96 antibody, the immune response suppressant for an IL-17-producing cell comprising 0.5 to 50 mg, preferably 0.7 mg to 10 mg, more preferably 1 mg to 5 mg, of the anti-CD96 antibody per kg body weight in a single dose can be administered once to several times a day. The total number of doses and administration frequency are not particularly limited and can be appropriately selected depending on, for example, the type or severity of symptoms of the disease or the pathological condition involving the immune response of an IL-17-producing cell, a recipient (e.g., age, sex, and body weight), and an administration route. Only one administration may be performed, daily continuous administration may be performed, or administration may be performed over one to several weeks at intervals of several days.

The immune response suppressant for an IL-17-producing cell may be administered as it is to a living body and is preferably administered as a medicament comprising an effective amount of the immune response suppressant for an IL-17-producing cell blended with a pharmaceutically acceptable carrier. An administration method is not particularly limited and can be oral or parenteral administration. The administration method is preferably parenteral administration, more preferably intraperitoneal injection or intravenous injection.

### <Medicament for treating or preventing disease or pathological condition involving immune response of IL-17-producing cell>

The medicament for treating or preventing a disease or a pathological condition involving the immune response of an IL-17-producing cell according to the present invention is not particularly limited as long as it comprises the immune response suppressant for an IL-17-producing cell. A pharmaceutically acceptable carrier may be contained therein, in addition to the immune response suppressant for an IL-17-producing cell.

The disease or the pathological condition involving the immune response of an IL-17-producing cell is not particularly limited as long as the disease or the pathological condition involves the immune response of an IL-17-producing cell. Psoriasis, rheumatoid arthritis, atopic dermatitis, multiple sclerosis, cerebral infarction, nephritis, lung injury, pulmonary fibrosis or skin tumor is preferred, psoriasis or multiple sclerosis is more preferred, and psoriasis is further preferred.

The form of the medicament is not particularly limited and is, for example, an injection, an injectable filler, an oral agent (a tablet, a granule, a powder, a capsule (also including a soft capsule), a solution, and a suspension), or a topical product (e.g., a lotion, an emulsion, a patch, and an ointment). The medicament may be prepared in the form of a freeze-dried formulation or an aqueous solution.

The dose of the medicament is not particularly limited and can be appropriately selected depending on the type or severity of symptoms of the disease or the pathological condition involving the immune response of an IL-17-producing cell.

Examples of the pharmaceutically acceptable carrier include, but are not particularly limited to: phosphate, citrate, and other organic acid buffer solutions; antioxidants including ascorbic acid and methionine; preservatives such as octadecyl dimethyl benzyl ammonium chloride, hexamethonium chloride, benzalkonium chloride, benzethonium chloride, benzyl alcohol, alkyl paraben, catechol, resorcinol, cyclohexanol, 3-pentanol, and m-cresol; low-molecular polypeptides; proteins such as serum albumin, gelatin, and immunoglobulins; hydrophilic polymers such as polyvinylpyrrolidone; amino acids such as glycine, glutamine, asparagine, histidine, arginine, and lysine; saccharides such as glucose, mannose, sucrose, mannitol, trehalose, sorbitol, and dextrin; chelating agents such as EDTA; salt-forming counterions such as sodium; metal complexes; and nonionic surfactants such as polyhydric alcohols (e.g., polyethylene glycol (PEG)), polysorbate 20, and polysorbate 80.

The recipient of the medicament is not particularly limited and is preferably a mammal, more preferably a human.

One embodiment of the present invention is a method for suppressing the immune response of an IL-17-producing cell, comprising administering an effective amount of a substance that inhibits the binding of CD96 to at least one protein selected from CD155 and CD111 to a subject.

One embodiment of the present invention is a method for treating or preventing a disease or a pathological condition involving the immune response of an IL-17-producing cell, comprising administering an effective amount of a substance that inhibits the binding of CD96 to at least one protein selected from CD155 and CD111 to a subject.

One embodiment of the present invention is a method for treating or preventing psoriasis, rheumatoid arthritis, atopic dermatitis, multiple sclerosis, cerebral infarction, nephritis, lung injury, pulmonary fibrosis or skin tumor, comprising administering an effective amount of a substance that inhibits the binding of CD96 to at least one protein selected from CD155 and CD111 to a subject.

One embodiment of the present invention is a substance that inhibits the binding of CD96 to at least one protein selected from CD155 and CD111 for use in the immune response suppression of an IL-17-producing cell.

One embodiment of the present invention is a substance that inhibits the binding of CD96 to at least one protein selected from CD155 and CD111 for treating or preventing a disease or a pathological condition involving the immune response of an IL-17-producing cell.

One embodiment of the present invention is a substance that inhibits the binding of CD96 to at least one protein selected from CD155 and CD111 for treating or preventing psoriasis, rheumatoid arthritis, atopic dermatitis, multiple sclerosis, cerebral infarction, nephritis, lung injury, pulmonary fibrosis or skin tumor.

### <Method comprising step (A)>

The third aspect of the present invention is a method comprising a step (A). In the present invention, the step (A) is the step of culturing at least one of a γδT cell and a CD4-positive T cell together with IL-23, an anti-CD3 antibody capable of stimulating a TCR/CD3 complex and an anti-CD96 antibody capable of stimulating CD96.

In other words, the method comprising the step (A) of the present invention may be a method for treating at least one of a γδT cell and a CD4-positive T cell. The purpose or application of the method comprising the step (A) is not particularly limited. The method can be used in, for example, the elucidation of the immune response mechanism of γδT cells or CD4-positive T cells, or the screening and evaluation of a medicament having an effect of inhibiting, suppressing, promoting, or potentiating the immune response of γδT cells or CD4-positive T cells, because at least one of a γδT cell and a CD4-positive T cell is stimulated with IL-23, an anti-CD3 antibody capable of stimulating a TCR/CD3 complex and an anti-CD96 antibody capable of stimulating CD96 to induce the immune response in the γδT cell or the CD4-positive T cell.

One embodiment of the present invention is a method for culturing a cell, comprising a step (A) of culturing at least one of a γδT cell and a CD4-positive T cell together with IL-23, an anti-CD3 antibody capable of stimulating a TCR/CD3 complex and an anti-CD96 antibody capable of stimulating CD96.

The step (A) permits induction of the immune response of γδT cells or CD4-positive T cells, activation of γδT cells or CD4-positive T cells, and induction of the IL-17 production of γδT cells or CD4-positive T cells.

Thus, the step (A) can also be used as a method for inducing the immune response of a γδT cell or a CD4-positive T cell, a method for activating a γδT cell or a CD4-positive T cell, or a method for inducing the IL-17 production of a γδT cell or a CD4-positive T cell.

The method for inducing the immune response of a γδT cell or a CD4-positive T cell can evaluate the presence or absence of induction of the immune response, for example, by using the production of a cytokine such as IL-17, IL-22, TNF, or IFN-γ as an index, as mentioned later.

The method for activating a γδT cell or a CD4-positive T cell can evaluate the presence or absence of the activation, for example, by using an activation marker such as CD69 or CD25 as an index, as mentioned later.

The method for inducing the IL-17 production of a γδT cell or a CD4-positive T cell can evaluate the presence or absence of the induction of IL-17 production, for example, by using the amount of IL-17 produced or the activity of IL-17 as an index, as mentioned later.

IL-23 is a cytokine that is known to be produced by antigen-presenting cells and to induce the immune response of γδT cells. IL-23R and IL-12Rβ1 are known as receptors of IL-23. In the method comprising the step (A), the IL-23 is added in order to induce the immune response of γδT cells or CD4-positive T cells. The origin of the IL-23 is not limited as long as it is the IL-23 protein. A protein purified from a living body may be used, or a recombinant protein derived from *E. coli,* yeasts, plant cells, mammalian cells, or insect cells may be used. The full-length IL-23 or a portion thereof may be used as long as it can induce the immune response of γδT cells or CD4-positive T cells. There is no limitation on, for example, the presence or absence of posttranslational modification or glycosylation or the presence or absence of a carrier. The IL-23 is preferably a recombinant protein, more preferably a mammalian cell-derived or insect cell-derived recombinant protein, because the immune response of γδT cells or CD4-positive T cells can be efficiently induced.

The final concentration of the IL-23 in the culture system is preferably 0.001 to 20 ng/ml, more preferably 0.01 to 1 ng/ml.

The anti-CD3 antibody capable of stimulating a TCR/CD3 complex is used for stimulating a TCR/CD3 complex on T cell surface. The anti-CD3 antibody capable of stimulating a TCR/CD3 complex is not particularly limited as long as it can stimulate a TCR/CD3 complex on T cell surface. Examples thereof include immobilized anti-CD3 antibodies and agonistic (stimulatory) anti-CD3 antibodies. An immobilized anti-CD3 antibody is preferred because of easy obtainment. When the immobilized anti-CD3 antibody is added to cells, CD3 is cross-linked by the antibody so that signals from CD3 enter the cells, resulting in the transduction of signals similar to signals of antigen recognition by TCR into T cells.

The anti-CD3 antibody is immobilized and thereby serves as the anti-CD3 antibody capable of stimulating a TCR/CD3 complex. Thus, the anti-CD3 antibody for use in immobilization is not particularly limited as long as it can specifically bind to a TCR/CD3 complex on T cell surface. Various anti-CD3 antibodies such as NO. 40-0031 manufactured by TONBO/Cytek Biosciences, Inc. can be used. According to the animal species from which γδT cells are derived, an anti-CD3 antibody that binds to a TCR/CD3 complex of the animal species can be selected.

The concentration of the anti-CD3 antibody for use in immobilization is preferably 1 ng/ml to 10 µg/ml, more preferably 10 ng/ml to 1 µg/ml.

The anti-CD96 antibody capable of stimulating CD96 is used for stimulating CD96 on T cell surface. The anti-CD96 antibody capable of stimulating CD96 is not particularly limited as long as it can stimulate CD96 on T cell surface. Examples thereof include immobilized anti-CD96 antibodies and agonistic (stimulatory) anti-CD96 antibodies. An immobilized anti-CD96 antibody is preferred because of easy obtainment. When the immobilized anti-CD96 antibody is added to cells, CD96 is cross-linked by the antibody so that signals from CD96 enter the cells, resulting in the transduction of signals similar to those of the binding of the natural ligand CD155 on cell surface to CD96 into T cells.

The anti-CD96 antibody is immobilized and thereby serves as the anti-CD96 antibody capable of stimulating CD96. Thus, the anti-CD96 antibody for use in immobilization is not particularly limited as long as it can specifically bind to CD96 on cell surface. Various anti-CD96 antibodies can be used. Examples thereof include eBioscience(TM) rat anti-mouse CD96 antibody (manufactured by Invitrogen Corp., NO. 16-0960-82), an anti-human CD96 antibody (clone: NK92.39, manufactured by BioLegend, Inc., NO. 338402), and TX111.2. According to the animal species from which γδT cells are derived, an anti-CD96 antibody that binds to CD96 of the animal species can be selected.

The concentration of the anti-CD96 antibody for use in immobilization is preferably 100 ng/ml to 1 mg/ml, more preferably 1 µg/ml to 200 µg/ml.

The solid phase on which the anti-CD3 antibody or the anti-CD96 antibody is immobilized is not particularly limited and is, for example, a plate, a dish, a bag, a membrane, or beads. A plate is preferred, and a plate having a plurality of wells is more preferred, because of easy immobilization. The material of the solid phase is not particularly limited, and, for example, glass or polystyrene can be suitably used. The solid phase on which the anti-CD3 antibody or the anti-CD96 antibody is immobilized is preferably a polystyrene plate because of efficient immobilization and easy obtainment.

The solid phase surface may be untreated or may be treated so as to become hydrophobic or hydrophilic. A functional group such as a CHOH group, a >C=O group, a -COOH group, a -NH₂ group, or a >NH group may be introduced thereto.

A method for immobilizing the anti-CD3 antibody or the anti-CD96 antibody is not particularly limited, and various methods known in the art can be used. The antibody can be immobilized onto the solid phase by use of, for example, electrostatic interaction, hydrophobic interaction (physical adsorption), a hydrogen bond, charge transfer, or a covalent bond. Hydrophobic interaction is preferred because of convenient operation. Immobilization that exploits the hydrophobic interaction can be performed, for example, by adding an antibody solution to a plate, incubating it for a given time, and then discarding the antibody solution, followed by washing.

The antibody solution can be used in an amount sufficient for covering solid phase surface, and is preferably added at 50 to 200 µl/well, for example, when the solid phase is a 96-well plate.

The method comprising the step (A) may optionally comprise a step (D) of evaluating the immune response of the γδT cell or the CD4-positive T cell after performing the step (A). The immune response of the γδT cell or the CD4-positive T cell means a reaction that occurs in γδT cells or CD4-positive T cells among reactions that are performed in order to eliminate exogenous or endogenous foreign substance in specific response thereto, and may be ascribable to natural immunity or acquired immunity. The immune response of the γδT cell or the CD4-positive T cell is, for example, cytokine production. Examples thereof include the production of IL-17, IL-22, TNF, and IFN-γ. IL-17 production is preferred. Specifically, the method comprising the step (A) preferably comprises a step (B) of evaluating IL-17 after performing the step (A).

The method comprising the step (A) may be performed *in vivo* or performed *in vitro* and is preferably performed *in vitro.*

### <Step (B)>

The step (B) is an optional step that may be performed after the step (A), and is the step of evaluating IL-17. The state, particularly, immune response, of γδT cells or CD4-positive T cells can be evaluated by evaluating IL-17.

Thus, one exemplary embodiment of the present invention is a method comprising:
step (A): the step of culturing at least one of a γδT cell and a CD4-positive T cell together with IL-23, an anti-CD3 antibody capable of stimulating a TCR/CD3 complex and an anti-CD96 antibody capable of stimulating CD96; and
step (B): the step of evaluating IL-17 derived from the at least one of a γδT cell and a CD4-positive T cell subjected to the step (A).

The method can evaluate the immune response of a γδT cell or a CD4-positive T cell and evaluate the activation of a γδT cell or a CD4-positive T cell.

Thus, the method can be used as a method for evaluating the immune response of a γδT cell or a CD4-positive T cell and a method for evaluating the activation of a γδT cell or a CD4-positive T cell.

A method for evaluating IL-17 is not particularly limited as long as the method can evaluate IL-17. Examples thereof include a method of measuring the amount of IL-17 produced and a method of measuring the activity of IL-17. The IL-17 evaluation may be the evaluation of IL-17 derived from the cell subjected to the step (A) and may be the evaluation of intracellular IL-17 or may be the evaluation of IL-17 released to the outside of the cell, for example, present in a cell culture supernatant. The method for evaluating IL-17 is preferably a method of measuring the amount of IL-17 produced because there are many reports on its relation to diseases. Examples of the measurement of the amount of IL-17 produced include IL-17 mRNA level measurement by quantitative PCR, IL-17 protein level measurement by Western blotting or ELISA, and intracellular IL-17 protein level measurement using a flow cytometer. IL-17 mRNA level measurement by quantitative PCR is preferred because more upstream change in IL-17 production can be determined. Forward: 5'-TTTAACTCCCTTGGCGCAAAA-3' (SEQ ID NO: 1) and reverse: 5'-CTTTCCCTCCGCATTGACAC-3' (SEQ ID NO: 2) are preferably used as primers for the IL-17 mRNA level measurement by quantitative PCR.

### <Method for evaluating medicament>

The method for evaluating a medicament (candidate substance) according to the fourth aspect of the present invention is a method for evaluating a medicament (candidate substance) for treating or preventing a disease or a pathological condition involving the immune response of an IL-17-producing cell, comprising a method comprising a step (A) and then a step (B). The method for evaluating a medicament (candidate substance) according to the present invention can evaluate an effect of inhibiting, suppressing, promoting, or potentiating the immune response of IL-17-producing cells, possessed by the medicament (candidate substance). The method for evaluating a medicament (candidate substance) according to the present invention has a step (C) of adding the medicament (candidate substance) to the γδT cell or the CD4-positive T cell. The order of the step (C) is usually before the step (A), at the same time with the step (A), or after the step (A) and before the step (B) .

Thus, one exemplary embodiment of the present invention is a method for evaluating a medicament candidate substance for treating or preventing a disease or a pathological condition involving the immune response of an IL-17-producing cell, comprising:
step (A): the step of culturing at least one of a γδT cell and a CD4-positive T cell together with IL-23, an anti-CD3 antibody capable of stimulating a TCR/CD3 complex and an anti-CD96 antibody capable of stimulating CD96;
step (B): the step of evaluating IL-17 derived from the at least one of a γδT cell and a CD4-positive T cell subjected to the step (A); and
step (C): the step of adding the medicament candidate substance to the γδT cell or the CD4-positive T cell before the step (A), at the same time with the step (A), or after the step (A) and before the step (B).

In the method for evaluating a medicament according to the present invention, the step (A) and the step (B) are performed in the presence of and in the absence of the candidate substance, whereby the candidate substance can be confirmed to be effective for treating or preventing the disease or the pathological condition involving the immune response of an IL-17-producing cell when the amount of IL-17 produced in the presence of the candidate substance is smaller than that in the absence of the candidate substance.

The method for evaluating a medicament (candidate substance) according to the present invention may be a method for screening for a medicament (candidate substance).

Thus, one exemplary embodiment of the present invention is a method for screening for a medicament candidate substance for treating or preventing a disease or a pathological condition involving the immune response of an IL-17-producing cell, comprising:
step (A): the step of culturing at least one of a γδT cell and a CD4-positive T cell together with IL-23, an anti-CD3 antibody capable of stimulating a TCR/CD3 complex and an anti-CD96 antibody capable of stimulating CD96;
step (B): the step of evaluating IL-17 derived from the at least one of a γδT cell and a CD4-positive T cell subjected to the step (A); and
step (C): the step of adding the medicament candidate substance to the γδT cell or the CD4-positive T cell before the step (A), at the same time with the step (A), or after the step (A) and before the step (B).

In the method for screening for a medicament (candidate substance) according to the present invention, the step (A) and the step (B) are performed in the presence of and in the absence of the medicament (candidate substance), whereby the medicament (candidate substance) can be selected as a candidate of the medicament for treating or preventing a disease or a pathological condition involving the immune response of an IL-17-producing cell when the amount of IL-17 produced in the presence of the medicament (candidate substance) is smaller than that in the absence of the medicament (candidate substance). For example, the medicament (candidate substance) can be selected as a candidate of the medicament for treating or preventing a disease or a pathological condition involving the immune response of an IL-17-producing cell when the amount of IL-17 produced in the presence of the medicament (candidate substance) is 10%, 20%, 30%, 40%, 50%, 60%, 70%, or 80% or less of the amount of IL-17 produced in the absence of the medicament (candidate substance). The medicament (candidate substance) is preferably selected as a candidate of the medicament for treating or preventing a disease or a pathological condition involving the immune response of an IL-17-producing cell when the amount of IL-17 produced in the presence of the medicament (candidate substance) is 10%, 20%, 30%, 40%, or 50% or less of the amount of IL-17 produced in the absence of the medicament (candidate substance).

Examples of the medicament (candidate substance) include, but are not particularly limited to, low-molecular compounds, proteins, polypeptides, polysaccharides, and nucleic acids. The medicament (candidate substance) may be a novel substance or a substance known in the art.

The disease or the pathological condition involving the immune response of an IL-17-producing cell is preferably psoriasis, rheumatoid arthritis, atopic dermatitis, multiple sclerosis, cerebral infarction, nephritis, lung injury, pulmonary fibrosis or skin tumor, more preferably psoriasis.

### <Method for producing IL-17>

The method for producing IL-17 according to the fifth aspect of the present invention comprises a step (A) of culturing at least one of a γδT cell and a CD4-positive T cell together with IL-23, an anti-CD3 antibody capable of stimulating a TCR/CD3 complex and an anti-CD96 antibody capable of stimulating CD96. IL-17 production in γδT cells or CD4-positive T cells is induced by stimulating the γδT cells or the CD4-positive T cells with IL-23, an anti-CD3 antibody capable of stimulating a TCR/CD3 complex and an anti-CD96 antibody capable of stimulating CD96.

The method for producing IL-17 according to the fifth aspect of the present invention can also be regarded as a method for manufacturing IL-17, comprising a step (A) of culturing at least one of a γδT cell and a CD4-positive T cell together with IL-23, an anti-CD3 antibody capable of stimulating a TCR/CD3 complex and an anti-CD96 antibody capable of stimulating CD96.

The application of IL-17 obtained by the method for producing IL-17 is not particularly limited, and it may be used after being isolated by, for example, affinity chromatography or immune precipitation. The γδT cell or the CD4-positive T cell allowed to produce IL-17 may be used in, for example, the screening and evaluation of a medicament having an effect of inhibiting, suppressing, promoting, or potentiating IL-17 production.

The method for producing IL-17 may be performed *in vivo* or performed *in vitro* and is preferably performed *in vitro.*

### Examples

Next, the present invention will be described in more detail with reference to Examples. However, the present invention is not limited by these.

### [Example 1]

### <<Method>>

### <Imiquimod-induced psoriasis model>

This model is generally used as a pathological condition model of psoriasis in which inflammation is induced by applying imiquimod (IMQ) to the skin of the shaved mouse back for 3 to 6 consecutive days. Pathological conditions characteristic of psoriasis, such as epidermal hyperplasia, parakeratosis, and the infiltration of neutrophils and mononuclear cells into the dermis, are observed, and the mechanism thereof has been reported as given below. IMQ activates dendritic cells via TLR7 so that IL-23 is produced. The IL-23 activates γδT cells via IL-23R/IL-12Rβ1 on the γδT cells, which in turn produce IL-17 and IL-22. The IL-17 and the IL-22 promote the migration of neutrophils to an affected part, inducing erythema, scaling, and skin hyperplasia.

Six to eight weeks old male C57BL/6J (wild-type mice) or CD96-deficient mice were used and divided into an imiquimod group (IMQ) and a naive group each involving four mice. The CD96-deficient mice were prepared by the genome editing technique (CRISPR/Cas9). AATAGAGACAAATCGGACTCTGG (SEQ ID NO: 3) was used as a target sequence. The backs of the mice were shaved, and 5% imiquimod cream (BESELNA Cream, manufactured by Mochida Pharmaceutical Co., Ltd.) was applied at 31.25 mg/dose/mouse once a day to the imiquimod group. Vaseline was applied instead of 5% imiquimod cream to the naive group. The start day of imiquimod cream or Vaseline application was defined as day 0. The application was performed on days 0, 1 and 2, and analysis was conducted on day 3.

### (Evaluation of erythema and scaling)

Erythema and scaling of the back were scored on the basis of PASI (Psoriasis Area and Severity Index) which is utilized in the diagnosis of human psoriasis. The scores were evaluated on 5 scales: 0: none, 1: mild, 2: moderate, 3: severe, and 4: very severe.

### (Evaluation of skin thickness)

The skin of the mouse back was lifted, and the thickness was measured using Dial Thickness Gauge Model G (manufactured by Ozaki MFG. Co., Ltd.). The measurement value was divided by 2, and the resulting value was regarded as a skin thickness (µm). Measurements were performed at three locations per mouse, and a mean was calculated. An error was calculated using the value of each mouse, followed by a significant different test in each group.

### (Preparation and observation of tissue preparation)

On day 3 from the start of application, the skin of the mouse back was collected, fixed in formalin, and embedded in paraffin. A 10 µm thick tissue section was prepared and stained with HE (hematoxylin-eosin) to prepare a tissue preparation. The tissue preparation was observed under a microscope (BZ-X710, Keyence Corp.) and photographed. The thickness of the epidermis in the image was measured and regarded as an epidermal thickness (µm). Three preparations were prepared per mouse. The thicknesses of five points were measured per preparation, and a mean was calculated. An error was calculated using the value of each mouse, followed by a significant different test in each group.

### (The number of neutrophil in skin)

On day 3 from the start of application, 2 cm² of the skin of the mouse back was collected, and skin cells were separated therefrom using 1 mg/ml collagenase IV (manufactured by Sigma-Aldrich Co. LLC). Live cells were counted using ViaCount assay (manufactured by GE Healthcare Japan Corp.). The skin cells were stained using Brilliant Violet 421-rat anti-mouse CD45.2 antibody (manufactured by BioLegend, Inc.), APC/Cy7-rat anti-mouse/human CD11b antibody (manufactured by BioLegend, Inc.), and Alexa Fluor 700-rat anti-mouse Ly6G antibody (manufactured by BD Biosciences), and cells stained with all of these 3 types of antibodies were counted as neutrophils. A flow cytometer (LSRFortessa(TM), manufactured by Nippon Becton Dickinson Co., Ltd.) was used in analysis. The number of neutrophils was divided by the area of the collected skin, and the resulting value was regarded as the number of neutrophils in the skin.

### (Amount of IL-23 produced from skin-derived myeloid cell)

On day 3 from the start of application, 2 cm² of the skin of the mouse back was collected, and skin cells were separated therefrom using 1 mg/ml collagenase IV (manufactured by Sigma-Aldrich Co. LLC). Live cells were counted using ViaCount assay (manufactured by GE Healthcare Japan Corp.). The skin cells were stained using Brilliant Violet 421-rat anti-mouse CD45.2 antibody (manufactured by BioLegend, Inc.) and APC/Cy7-rat anti-mouse/human CD11b antibody (manufactured by BioLegend, Inc.), and cells stained with both the antibodies were sorted as myeloid cells using a cell sorter (FACSAria(TM), manufactured by Nippon Becton Dickinson Co., Ltd.).

These cells were recovered, and mRNA was extracted using ISOGEN (manufactured by Nippon Gene Co., Ltd.) in accordance with the product's protocol. cDNA was synthesized from the mRNA using High-Capacity cDNA Reverse Transcription Kit (manufactured by Applied Biosystems, Inc.) in accordance with the protocol. Quantitative PCR was performed using SYBR Green PCR Master Mix (manufactured by Invitrogen Corp.), and analysis was conducted with ABI 7500 fast (manufactured by Applied Biosystems, Inc.) to measure the expression level of IL-23. The primers used for IL-23 were forward: 5'-CAGCTCTCTCGGAATCTCTGC-3' (SEQ ID NO: 4) and reverse: 5'-GACCTTGGCGGATCCTTTGC-3' (SEQ ID NO: 5).

### (Expression level of IL-23 receptor on skin-derived γδT cell)

On day 3 from the start of application, 2 cm² of the skin of the mouse back was collected, and skin cells were separated therefrom using 1 mg/ml collagenase IV (manufactured by Sigma-Aldrich Co. LLC). Live cells were counted using ViaCount assay (manufactured by GE Healthcare Japan Corp.). The skin cells were stained using Brilliant Violet 421-rat anti-mouse CD45.2 antibody (manufactured by BioLegend, Inc.) and eBioscience(TM) FITC-hamster anti-mouse TCR GAMMA DELTA antibody (Catalog NO. 118106, manufactured by BioLegend, Inc.), and cells stained with both the antibodies were sorted as γδT cells using a cell sorter (FACSAria, manufactured by Nippon Becton Dickinson Co., Ltd.).

mRNA extraction, cDNA synthesis, and quantitative PCR were performed in the same manner as above to measure the amounts of IL-23R and IL-12Rβ1 produced. The primers used for IL-23R were forward: 5'-GCAACATGACATGCACCTGG-3' (SEQ ID NO: 6) and reverse: 5'-GACAGCTTGGACCCATACCA-3' (SEQ ID NO: 7). The primers used for IL-12Rβ1 were forward: 5'-ATGGCTGCTGCGTTGAGAA-3' (SEQ ID NO: 8) and reverse: 5'-AGCACTCATAGTCTGTCTTGGA-3' (SEQ ID NO: 9).

### (Amounts of IL-17 and IL-22 produced in mouse skin-derived γδT cell)

The preparation of γδT cells, mRNA extraction, cDNA synthesis, and quantitative PCR were performed in the same manner as above to measure the amounts of IL-17 and IL-22 produced. The primers used for IL-17 were the same as above. The primers used for IL-22 were forward: 5'-TTTCCTGACCAAACTCAGCA-3' (SEQ ID NO: 10) and reverse: 5'-CTGGATGTTCTGGTCGTCAC-3' (SEQ ID NO: 11).

### (Ratio of IL-17-positive cell to mouse skin-derived γδT cell)

On day 3 from the start of application, 2 cm² of the skin of the mouse back was collected, and skin cells were separated therefrom using 1 mg/ml collagenase IV (manufactured by Sigma-Aldrich Co. LLC). Live cells were counted using ViaCount assay (manufactured by GE Healthcare Japan Corp.).

The collected cells were cultured at 37°C for 2 hours in a 5% CO₂ environment using a RPMI-1640 medium (R-8758, manufactured by Sigma-Aldrich Co. LLC) that contained 1/2000 of GolgiStop(TM) (manufactured by BD Biosciences) and was supplemented with 5% FBS. IL-17 produced by γδT cells was stained by staining with Brilliant Violet 421-rat anti-mouse CD45.2 antibody (manufactured by BioLegend, Inc.) and eBioscience(TM) FITC-hamster anti-mouse TCR GAMMA DELTA antibody (Catalog NO. 118106, manufactured by BioLegend, Inc.) and subsequent fixation with eBioscience(TM) Foxp3/Transcription Factor Fixation/Permeabilization Concentrate and Diluent (manufactured by Invitrogen Corp.), followed by staining with PE-rat anti-mouse IL17A antibody (BD Biosciences). Analysis was conducted using a flow cytometer (LSRFortessa(TM), manufactured by Nippon Becton Dickinson Co., Ltd.). Cells stained with the PE-rat anti-mouse IL17A antibody were regarded as being IL-17-positive, and divided by the number of γδT cells to calculate the ratio of IL-17-positive cells.

### <IL-17 production in mouse spleen-derived γδT cell by stimulation with IL-23 and CD96>

The spleen was collected from each wild-type mouse (C57BL/6, 6 to 8 weeks old male, distributor: CLEA Japan, Inc.) and ground using a glass slide to separate cells. Live cells were counted using ViaCount assay (manufactured by GE Healthcare Japan Corp.). The spleen cells were stained using Brilliant Violet 421-rat anti-mouse CD45.2 antibody (manufactured by BioLegend, Inc.) and eBioscience(TM) FITC-hamster anti-mouse TCR GAMMA DELTA antibody (manufactured by Invitrogen Corp., Catalog NO. 11-5811-82), and cells stained with both the antibodies were sorted as γδT cells using a cell sorter (FACSAria, manufactured by Nippon Becton Dickinson Co., Ltd.).

First, an antibody was immobilized onto a plate. Specifically, eBioscience(TM) rat anti-mouse CD96 antibody (manufactured by Invitrogen Corp., NO. 16-0960-82, 20 µg/ml) or rat IgG2a (manufactured by BD Bioscience, NO. 554687, 20 µg/ml; also referred to as rG2a) as a control was added at 100 µL/well to a 96-well plate (manufactured by Corning Inc., NO. 3596) and then incubated at 37°C for 2 hours. Then, the antibody solution was discarded, and the plate was washed once with PBS.

The γδT cells were inoculated at 15000 cells/well to this plate, and recombinant mouse IL-23 (manufactured by R&D systems, Inc., NO. 1887-ML-010, final concentration: 0.1 ng/ml) was added to RPMI-1640 medium (manufactured by Sigma-Aldrich Co. LLC, R-8758) supplemented with 10% FBS and recombinant mouse IL-2 (manufactured by BD Bioscience, NO. 55069, final concentration: 2 ng/ml), followed by culture at 37°C for 18 hours in a 5% CO₂ environment.

mRNA extraction, cDNA synthesis, and quantitative PCR were performed in the same manner as above to measure the amount of IL-17 produced. The primers used for IL-17 were the same as above.

### <IL-17 production in mouse spleen-derived γδT cell by stimulation with IL-23, CD96, and CD3>

The same method as in the foregoing section "IL-17 production in mouse spleen-derived γδT cell by stimulation with IL-23 and CD96" was performed. For a group supplemented with CD3 signals, an anti-CD3 antibody (manufactured by TONBO/Cytek Biosciences, Inc., NO. 40-0031, final concentration: 0.1 µg/ml) was immobilized onto a plate. The immobilization was performed in the same manner as in the anti-CD96 antibody.

### (Activation of mouse spleen-derived γδT cell)

The same method as in the foregoing section "IL-17 production γδT cell activation in spleen-derived γδT cell by stimulation with IL-23, CD96, and CD3" was performed. The activation of γδT cells was evaluated by using CD69 and CD25 as activation markers. The γδT cells were stained with APChamster anti-mouse CD69 antibody (manufactured by BioLegend, Inc.) and PE-rat anti-mouse CD25 antibody (manufactured by BioLegend, Inc.), and a flow cytometer (LSRFortessa(TM), manufactured by Nippon Becton Dickinson Co., Ltd.) was used in analysis.

### (Human PBMC-derived γδT cell)

Written informed consent was obtained from test subjects before a test in accordance with the Helsinki Declaration. The present test was conducted with the approval of the ethics committee of the School of Medicine and Health Sciences, University of Tsukuba. The test subjects were volunteered healthy persons. 60 ml of blood was collected from each test subject, and peripheral blood mononuclear cells (PBMCs) were separated therefrom using Lymphoprep (manufactured by Stemcell technologies Inc., NO. 07801). Live cells were counted using ViaCount assay (manufactured by GE Healthcare Japan Corp.). Vδ2 γδT cells in the PBMCs were stained with PE-anti-hTCRγδ (manufactured by eBiosciences, NO. 12-9959-42) and BV605-anti-hVδ2 (manufactured by BD Biosciences, NO. 743751), sorted using a cell sorter (FACSAria, manufactured by Nippon Becton Dickinson Co., Ltd.), and subjected as human PBMC-derived γδT cells to the experiment.

### (Human PBMC-derived CD4+ T cell)

10 ml of blood was collected from each volunteered healthy person, and peripheral blood mononuclear cells (PBMCs) were separated therefrom using Lymphoprep (manufactured by Stemcell technologies Inc., NO. 07801). Live cells were counted using ViaCount assay (manufactured by GE Healthcare Japan Corp.). Cells isolated using Human CD4 microbeads (manufactured by Miltenyi Biotec GmbH, NO. 130-045-101) were subjected as human PBMC-derived CD4+ T cells to the experiment.

### <Expression of CD96 in human PBMC-derived γδT cell and CD4+ T cell>

PE-anti-hCD96 (manufactured by BioLegend, Inc., NO. 338406) or PE-mouse IgG1 isotype control (manufactured by BD Biosciences, NO. 555749) was added to human PBMC-derived γδT cells and incubated, followed by analysis with a flow cytometer. Human PBMC-derived CD4+ T cells were experimented in the same manner.

### <IL-17 production in human PBMC-derived γδT cell>

Human PBMC-derived γδT cells were inoculated to a plate with an anti-CD3 antibody and a CD96 antibody immobilized thereon, and stimulated for 48 hours. Then, IL-17 production by the γδT cells was evaluated. The immobilization of the antibodies was performed in accordance with the foregoing section "IL-17 production in spleen-derived γδT cell by stimulation with IL-23 and CD96". The antibodies used in immobilization were 100 ng/ml Anti-hCD3 (manufactured by BD Biosciences, NO. 555336) and 20 µg/ml Anti-hCD96 (manufactured by BioLegend, Inc., NO. 338404).

The γδT cells were inoculated at 250000 cells/well and cultured at 37°C for 48 hours in a 5% CO₂ environment using 10% FBS and RPMI-1640 medium (manufactured by Sigma-Aldrich Co. LLC, R-8758) supplemented with Recombinant Human TGF-beta 1 (manufactured by R&D systems, Inc., NO. 240-B, final concentration: 50 ng/ml), Recombinant Human IL-1β (manufactured by R&D systems, Inc., NO. 201-LB, final concentration: 10 ng/ml), Recombinant Human IL-23 (manufactured by R&D systems, Inc., NO. 1290-IL, final concentration: 20 ng/ml), Recombinant Human IL-6 (manufactured by R&D systems, Inc., NO. 7270-IL, final concentration: 50 ng/ml), and Recombinant Human IL-12 (BD Pharmingen(TM), NO. 554613, final concentration: 10 ng/ml).

The IL-17A protein in the cell culture supernatant was measured using Human IL-17A Flex set (manufactured by BD Biosciences, NO. 560383).

### <Activation of human PBMC-derived γδT cell and human PBMC-derived CD4+ T cell>

A T cell enriched fraction containing γδT cells and CD4+ T cells was inoculated to a plate with an anti-CD3 antibody and a CD96 antibody immobilized thereon, and stimulated for 18 hours. Then, the activation of the γδT cells and the CD4+ T cells was evaluated by using CD69 and CD25 as activation markers. The immobilization of the antibodies was performed in accordance with the foregoing section "IL-17 production in spleen-derived γδT cell by stimulation with IL-23 and CD96". The antibodies used in immobilization were 100 ng/ml Anti-hCD3 (manufactured by BD Biosciences, NO. 555336) and 20 µg/ml Anti-hCD96 (manufactured by BioLegend, Inc., NO. 338404).

Specifically, PBMCs were bound with biotin-anti-hCD14 (manufactured by BioLegend, Inc., NO. 301826) and biotin-anti-hCD19 (manufactured by BD Biosciences, NO. 555411), and T cells were enriched by negative selection which selected cells expressing neither CD14 nor CD19 using streptavidin Microbeads (manufactured by Miltenyi Biotec GmbH, NO. 130-048-102). This T cell enriched fraction was inoculated at 500000 cells/well to the plate with the anti-CD3 antibody and the CD96 antibody immobilized thereon, and cultured at 37°C for 18 hours in a 5% CO₂ environment using 10% FBS and RPMI-1640 medium (manufactured by Sigma-Aldrich Co. LLC, NO. R-8758).

The cells thus cultured were analyzed with a flow cytometer. The γδT cells were stained with PE-anti-hTCRγδ (manufactured by BD Biosciences, NO. 555717), and the CD4+ T cells were stained with V500-anti-hCD4 (manufactured by BD Biosciences, NO. 560768). The activation markers were detected in their respective cells. The activation markers used were FITC-anti-hCD69 (manufactured by BD Biosciences, NO. 555530) and PE-Cy7-anti-hCD25 (manufactured by TONBO/Cytek Biosciences, Inc., 60-0259-T025).

### <IL-17 production in human PBMC-derived CD4+ T cell>

Operation was performed in accordance with the foregoing section "IL-17 production in human PBMC-derived γδT cell" using human PBMC-derived CD4+ T cells instead of the human PBMC-derived γδT cells. The stimulation time after inoculation of the human PBMC-derived CD4+ T cells to a plate with an anti-CD3 antibody and a CD96 antibody immobilized thereon was 96 hours.

### <Action of anti-CD96 neutralizing antibody on IL-17 production of human PBMC-derived CD4+ T cell>

Human PBMC-derived CD4+ T cells mixed in advance with an anti-CD96 neutralizing antibody were inoculated to a plate with an anti-CD3 antibody and hCD155-Fc (chimeric protein of a CD96 natural ligand CD155 and Fc) immobilized thereon, and stimulated for 6 hours. Then, IL-17 production by the CD4+ T cells was evaluated.

293gp cells were transfected with pMX plasmid having an insert of the full-length human CD155 gene on the N-terminal side of the human IgG Fc gene, to obtain a chimeric protein of full-length human CD155 fused with human IgG Fc (hCD155-Fc). The hCD155-Fc was purified using protein-A agarose and then subjected to the subsequent experiment.

DOTAP (1,2-dioleoyl-3-trimethylammoniumpropane, monochloride, manufactured by Cayman Chemical Company, NO. 15110) was added at a final concentration of 1 mg/ml to a 96-well plate (manufactured by Corning Inc., NO. 3596) and left standing for 15 minutes. Then, the solution was discarded, and the plate was washed twice with PBS. 100 ng/ml Anti-hCD3 (manufactured by BD Biosciences, NO. 555336) and 10 µg/ml hCD155-Fc were added at 100 µL/well to this plate and incubated overnight at 4°C. Then, the solution was discarded, and the plate was washed once with PBS to immobilize the anti-CD3 antibody and the hCD155-Fc. The DOTAP, a cationic lipid, was used for allowing the antibody and the Fc protein to stand firmly in the plate, and efficiently introducing stimulation. CD155 was used as the chimeric protein CD155-Fc with Fc for efficient immobilization onto the plate. The immobilized hCD155-Fc can be stimulated with CD96 *in vitro.*

An anti-human CD96 neutralizing antibody (clone: NK92.39, manufactured by BioLegend, Inc., NO. 338402) was added at 10 µg/2 × 10⁵ cells to healthy person-derived human PBMC-derived CD4+ T cells so that the anti-human CD96 antibody was bound with CD96 of the CD4+ T cells. CD4+ T cells supplemented with PBS instead of the anti-human CD96 neutralizing antibody were used as a control.

The CD4+ T cells mixed in advance with the anti-human CD96 neutralizing antibody were inoculated to the plate with the anti-CD3 antibody and the hCD155-Fc immobilized thereon, and the cells were cultured for 6 hours in accordance with the foregoing section "IL-17 production in human PBMC-derived γδT cell".

The IL-17A protein in the cell culture supernatant was measured using Human IL-17A Flex set (manufactured by BD Biosciences, NO. 560383).

### <Establishment of anti-CD96 neutralizing antibody TX111.2>

293gp cells were cotransfected with pMX plasmid having an insert of cDNA of the mouse CD96 gene and a retroviral envelope vector (VSV-G) to obtain recombinant retrovirus. A mouse lymphoma cell line BW5147.3 (hereinafter, referred to as BW cells) was transduced with this virus to obtain a stably mouse CD96-expressing cell line (hereinafter, referred to as CD96-BW cells) which strongly expressed full-length mouse CD96.

293gp cells were transfected with pMX plasmid having an insert of the mouse CD96 gene on the N-terminal side of the human IgG Fc gene to obtain a chimeric protein of the full-length mouse CD96 protein fused with human IgG Fc (mCD96-huIgGFc). The mCD96-huIgGFc was purified using protein-A agarose and then used in immunization.

A CD96-BW cell suspension (5 × 10⁷ cells/mL in PBS, 1 × 10⁷ cells/mouse) was intraperitoneally injected once to each CD96-deficient mouse at the start of immunization. One month later therefrom, mCD96-humanIgGFc (10 µg in PBS) was intraperitoneally injected thereto. The administration of mCD96-humanIgGFc was performed a total of five times at 1-month intervals. 3 days after final immunization, spleen cells were collected from the immunized mouse. The spleen cells were subjected to cell fusion with a mouse myeloma cell line SP2/0-Ag14 using polyethylene glycol to prepare hybridomas.

Hybridomas producing an antibody that specifically recognized mCD96 were screened for by FACS using the CD96-BW cells. An antibody produced by the obtained clone was designated as TX111.

The DNA sequences of the light chain and heavy chain variable regions of TX111 were determined by PCR. The detailed method is as described in Thomas Tiller et al., Journal of immunological methods, 350, 2009, 183-193.

CDR (complementarity determining region) 1 to CDR3 of the light chain and heavy chain variable regions of TX111 were determined by use of IgBLAST.

mRNAs encoding the heavy chain and light chain variable regions of the antibody were extracted from the hybridoma producing TX111. cDNAs of the heavy chain and light chain variable regions of the antibody were synthesized from the obtained mRNAs using reverse transcriptase.

These two cDNAs were respectively cloned into pCDNA(TM)3.4-TOPO vectors (manufactured by Invitrogen Corp.) having a DNA insert of Fc mutated such that the Fc moiety would not bind to an Fc receptor. 293F cells were transfected with these two vectors using ExpiFectamine(TM) 293 Transfection Kit (manufactured by Thermo Fisher Scientific Inc., NO. A14525). An antibody purified from a culture supernatant using HiTrap Protein G (manufactured by Merck KGaA, NO. GE17-0404-01) was designated as TX111.2.

### <Binding specificity of anti-mouse CD96 neutralizing antibody TX111.2>

10 ng of TX111.2 or 10 ng of Mouse IgG2b isotypic control (manufactured by TONBO/Cytek Biosciences, Inc., NO. 70-4732-U100) was added to 1 × 10⁵ CD96-BW cells and incubated on ice for 30 minutes. After washing with PBS, APC-anti-mIgG (manufactured by BioLegend, Inc., NO. 405308) was added thereto and incubated on ice for 20 minutes for staining, followed by analysis with a flow cytometer.

### <Competitive inhibition between anti-mouse CD96 neutralizing antibody TX111.2 and CD155>

293gp cells were transfected with pMX plasmid having an insert of the full-length mouse CD155 gene on the N-terminal side of the human IgG Fc gene to obtain a chimeric protein of full-length mouse CD155 fused with human IgG Fc (mCD155-huIgGFc). The mCD155-huIgGFc was purified using protein-A agarose and then used in the subsequent experiment.

1 µg of mCD155-huIgGFc was added to 1 × 10⁵ CD96-BW cells (T.F.) and incubated on ice for 30 minutes. After washing with PBS, 1000 ng, 100 ng, 10 ng, 1 ng, or 0.1 ng of TX111.2 was added thereto. After incubation at room temperature for 30 minutes, PE-anti-huIgG (manufactured by BioLegend, Inc., NO. 410708) was added thereto and incubated on ice for 20 minutes for staining, followed by analysis with a flow cytometer. The CD155-huIgGFc is a chimeric protein of mouse CD155 fused with Fc of human IgG.

### <Prophylactic administration experiment of anti-mouse CD96 neutralizing antibody to imiquimod-induced psoriasis mouse model>

The backs of C57BL/6J mice (wild-type, 8 weeks old, male) were shaved, and 5% imiquimod cream (BESELNA Cream, manufactured by Mochida Pharmaceutical Co., Ltd.) was applied thereto at 62.5 mg/dose/mouse once a day. Vaseline was applied instead of 5% imiquimod cream to a naive group. The start day of imiquimod cream application was defined as day 0. The application was performed on days 0, 1, 2 and 3, and analysis was conducted on days 3 and 4. On the day before the start day of imiquimod cream application (day -1), 100 µg of the anti-mouse CD96 neutralizing antibody TX111.2 was intravenously injected to a TX111.2 administration group (N = 5). A group given 100 µg of mouse IgG (cIg) instead of TX111.2 was used as a control antibody administration group (N = 5). The mouse IgG was produced by 293F cells and purified from a culture supernatant using HiTrap Protein G (manufactured by Merck KGaA, NO. GE17-0404-01).

On imiquimod application day 3, the skin of the mouse backs was photographed. On imiquimod application day 4, erythema, scaling and a skin thickness were evaluated, and skin tissue preparations were prepared. The skin tissue preparations were observed, and an epidermal thickness was measured.

### <Therapeutic administration experiment of anti-mouse CD96 neutralizing antibody to imiquimod-induced psoriasis mouse model>

The backs of C57BL/6J mice (wild-type, 8 weeks old, male) were shaved, and 5% imiquimod cream (BESELNA Cream, manufactured by Mochida Pharmaceutical Co., Ltd.) was applied thereto at 62.5 mg/dose/mouse once a day. Vaseline was applied instead of 5% imiquimod cream to a naive group. The start day of imiquimod cream application was defined as day 0. The application was performed on days 0, 1, 2, 3, 4 and 5, and analysis was conducted on days 1 and 6. On day 1 from the start of imiquimod cream application, 100 µg of the anti-mouse CD96 neutralizing antibody TX111.2 was intraperitoneally injected to a TX111.2 administration group (N = 6). A group given 100 µg of mouse IgG (cIg) instead of TX111.2 was used as a control antibody administration group (N = 6).

On imiquimod application day 6, the skin of the mouse backs was photographed. On imiquimod application days 1 and 6, erythema, scaling and a skin thickness were evaluated. On imiquimod application day 6, skin tissue preparations were prepared. The skin tissue preparations were observed, and an epidermal thickness was measured.

### <EAE model>

Experimental autoimmune encephalomyelitis (EAE) is an inflammatory demyelinating disease of the central nervous system. EAE is an autoimmune model in which T cells specific for myelin protein are induced by the inoculation of a laboratory animal with an encephalitogenic protein (peptide) such as a central nervous tissue-derived protein or peptide of another animal to develop autoimmune encephalomyelitis. Demyelination occurs by inoculation with the encephalitogenic protein (peptide) emulsified with an adjuvant such as a complete Freund's adjuvant. The presence of the adjuvant causes inflammatory reaction against such an encephalitogenic protein (peptide). In many experimental methods, pertussis toxin (PTX) is inoculated concurrently with the encephalitogenic protein (peptide) to disrupt the blood-brain barrier and allow immunocytes to invade the central nervous system. This inoculation develops multiple and sporadic small demyelinated regions in the brain and the spinal cord and eventually manifests a series of symptoms.

EAE shares many pathological conditions with, for example, multiple sclerosis (MS) and acute disseminated encephalomyelitis, and as such, is generally used in research on these pathological conditions or their therapy. In general, EAE also serves as a model of a T cell-mediated autoimmune disease.

### <Administration experiment of anti-mouse CD96 neutralizing antibody to EAE mouse model>

EAE models were prepared by inducing EAE in C57BL/6J mice (wild-type, 11 weeks old, female) using Hooke kit MOG₃₅-₅₅/CFA Emulsion PTX (Hooke laboratories Inc., EK-2110) in accordance with the protocol attached to the product. The encephalitogenic peptide used was myelin oligodendrocyte glycolipid (MOG). 0.1 mL of MOG/CFA (complete Freund's adjuvant) was subcutaneously administered to each of two locations (a total of 0.2 ml/mouse). Then, PTX was intraperitoneally administered at 0.1 mL/mouse (day 0). On the next day (day 1), PTX was intraperitoneally administered again at 0.1 mL/mouse. On the day before the start of MOG/CFA and PTX administration (day -1), 100 µg of the anti-mouse CD96 neutralizing antibody TX111.2 was intraperitoneally injected to a TX111.2 administration group (N = 5). After the start of MOG/CFA and PTX administration, 100 µg of TX111.2 was intraperitoneally administered a total of 10 times with a frequency of twice a week. A group given 100 µg of mouse IgG (cIg) instead of TX111.2 was used as a control antibody administration group (N = 5).

EAE pathological condition scores were evaluated every day after EAE induction on the scale of 0 to 5 on a 0.5 basis as given below in accordance with the protocol attached to the product. 0 means no abnormality, and a large number means higher severity.

0: No obvious changes in motor functions in comparison to non-immunized mice. When picked up by the base of tail, the tail has tension and is erect. Hind legs are usually spread apart. When the mouse is walking, there is no gait or head tilting.

0.5: Tip of tail is limp. When picked up by base of tail, the tail has tension except for the tip. Muscle straining is felt in the tail, while the tail continues to move.

1.0: Limp tail. When picked up by base of tail, instead of being erect, the whole tail drapes over finger. Hind legs are usually spread apart. No signs of tail movement are observed.

1.5: Limp tail and hind leg inhibition. When picked up by base of tail, the whole tail drapes over finger. When the mouse is dropped on a wire rack, at least one hind leg falls through consistently. Walking is very slightly wobbly.

2.0: A or B.

A: Limp tail and weakness of hind legs. When picked up by base of tail, the legs are not spread apart, but held closer together. When the mouse is observed walking, it has a clearly apparent wobbly walk. One foot may have toes dragging, but the other leg has no apparent inhibitions of movement.

B: Mouse appears to be at score 0.0, but there are obvious signs of head tilting when the walk is observed. The balance is poor.

2.5: A, B or C.

A: Limp tail and dragging of hind legs. Both hind legs have some movement, but both are dragging at the feet (mouse trips on hind feet).

B: No movement in one leg, or completely dragging one leg, but movement in the other leg.

C: EAE severity appears mild when the mouse is picked up (as score 0.0-1.5), but there is a strong head tilt that causes the mouse to occasionally fall over.

3.0: A, B, C or D.

A: Limp tail and complete paralysis of hind legs (most common).

B: Limp tail and almost complete paralysis of hind legs. One or both hind legs are able to paddle, but neither hind leg is able to move forward of the hip joint.

C: Limp tail with paralysis of one front and one hind leg.

D: All of the following a to d are satisfied.

a: Severe head tilting, b: Walking only along the edges of the cage, c: Pushing against the cage wall, and d: Spinning when picked up by base of tail.

3.5: A or B.

A: Limp tail and complete paralysis of hind legs. In addition, the mouse is moving around the cage, but when placed on its side, is unable to right itself. Hind legs are together on one side of body.

B: The mouse is moving around the cage, but the hind quarters are flat like a pancake, giving the appearance of a hump in the front quarters of the mouse.

4.0: Limp tail, complete hind leg and partial front leg paralysis. The mouse is minimally moving around the cage but appears alert and feeding. Often euthanasia is recommended after the mouse scores 4.0 for 2 days. However, with daily s.c. fluids most C57BL/6 mice may recover to 3.5 or 3.0. When the mouse is euthanized because of severe paralysis, a score of 5.0 is entered for that mouse for the rest of the experiment.

4.5: Complete hind leg and partial front leg paralysis. No movement around the cage. The mouse is not alert. The mouse has minimal movement in the front legs. The mouse barely responds to contact. Euthanasia is recommended. When the mouse is euthanized because of severe paralysis, a score of 5.0 is entered for that mouse for the rest of the experiment.

5.0: A, B or C.

A: The mouse is spontaneously rolling in the cage (euthanasia is recommended).

B: The mouse is found dead due to paralysis.

C: The mouse is euthanized due to severe paralysis.

### <<Results>>

### <Imiquimod-induced psoriasis models>

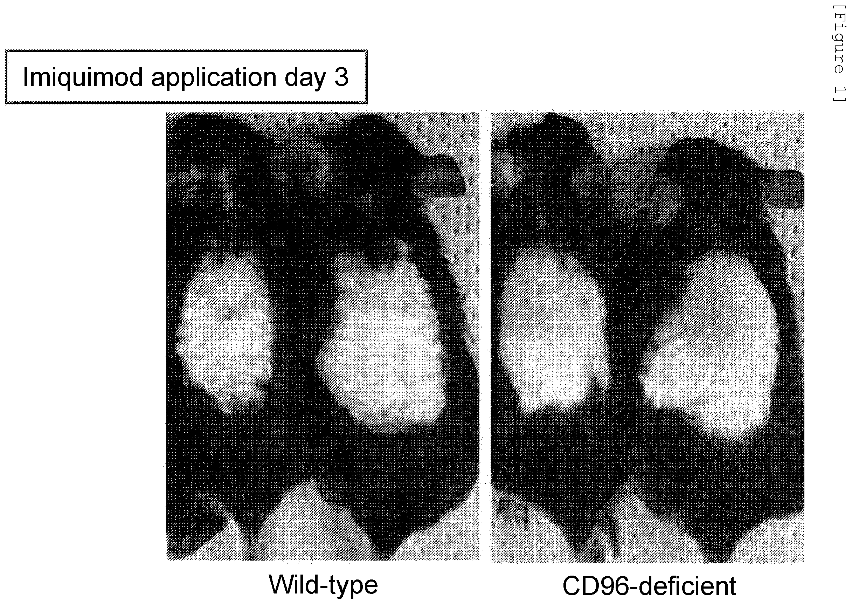

Figure 1 shows photographs of the backs on day 3 from the start of application of wild-type mice and CD96-deficient mice as imiquimod-induced psoriasis. In each photograph, the left mouse is a naive group, and the right mouse is an imiquimod (IMQ) group. In the wild-type mice, the skin became red with scaling confirmed in the imiquimod group compared with the naive group. In the CD96-deficient mice, the skin became red with scaling confirmed in the imiquimod group compared with the naive group, though the degrees thereof were reduced as compared with the wild-type mice.

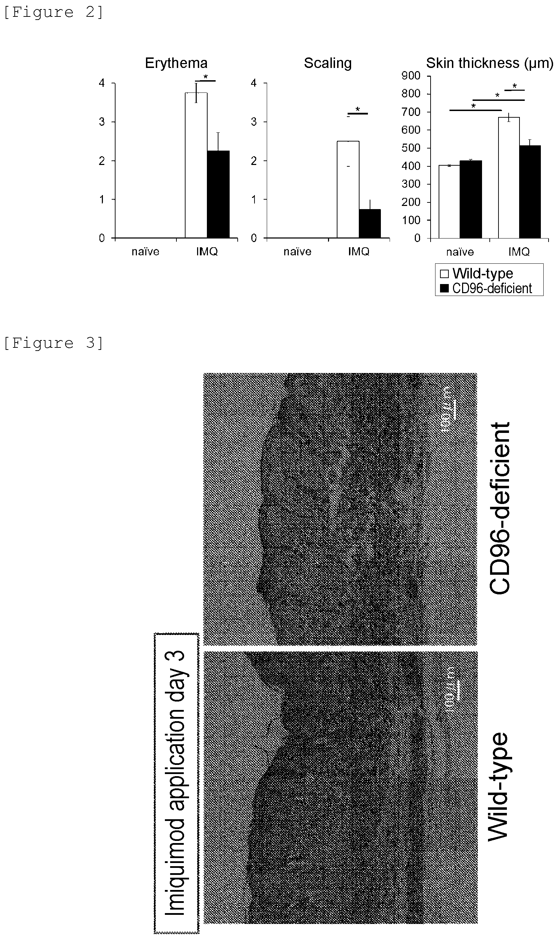

Figure 2 shows graphs in which erythema, scaling, and a skin thickness on day 3 from the start of application were compared between the wild-type mice and the CD96-deficient mice. The ordinates of the graphs about erythema and scaling depict an erythema score and a scaling score, respectively, and the ordinate of the graph about a skin thickness depicts a skin thickness (µm). Neither erythema nor scaling was seen in both the wild-type mice and the CD96-deficient mice in the naive group.

The erythema scores in the imiquimod group were higher for both the wild-type mice and the CD96-deficient mice than those in the naive group, whereas the erythema score of the CD96-deficient mice was significantly lower than that of the wild-type mice.

The scaling scores in the imiquimod group were higher for both the wild-type mice and the CD96-deficient mice than those in the naive group, whereas the scaling score of the CD96-deficient mice was significantly lower than that of the wild-type mice.

The skin in the imiquimod group was thicker for both the wild-type mice and the CD96-deficient mice than that in the naive group, whereas the skin of the CD96-deficient mice was significantly thinner than that of the wild-type mice.

These results indicated that the pathological conditions of erythema, scaling, and skin hyperplasia induced by imiquimod were reduced by CD96 deficiency.

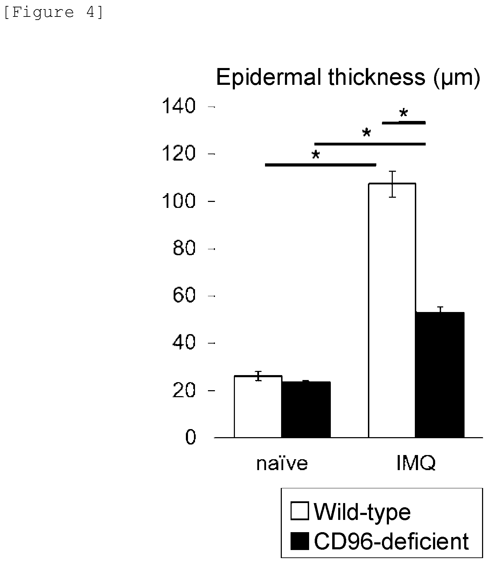

Figure 3 shows photographs of HE-stained tissue preparations of the mouse back skin on day 3 from the start of imiquimod application. Pathological conditions characteristic of psoriasis, such as epidermal hyperplasia, parakeratosis, and the infiltration of neutrophils and mononuclear cells into the dermis, were observed in the wild-type mice, whereas these pathological conditions were reduced in the CD96-deficient mice. An epidermal thickness was graphed in Figure 4. The epidermal thickness of the wild-type mice was approximately 4 times in the imiquimod group compared with the naive group, whereas the epidermal thickness of the CD96-deficient mice was approximately 2 times therein. Thus, epidermal hyperplasia caused by imiquimod was reduced in CD96-deficient mice compared to wild-type mice.

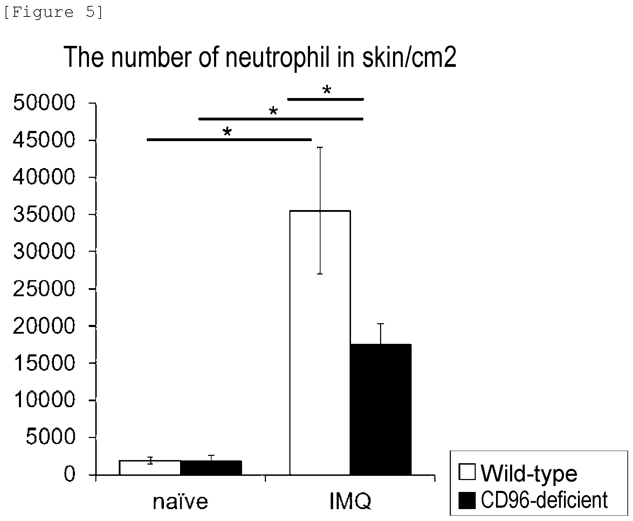

Figure 5 shows the number of neutrophils in the skin. The number of neutrophils in the wild-type mice was drastically increased in the imiquimod group compared with the naive group, whereas the effect of increasing the number of neutrophils in the skin by imiquimod was reduced in the CD96-deficient mice.

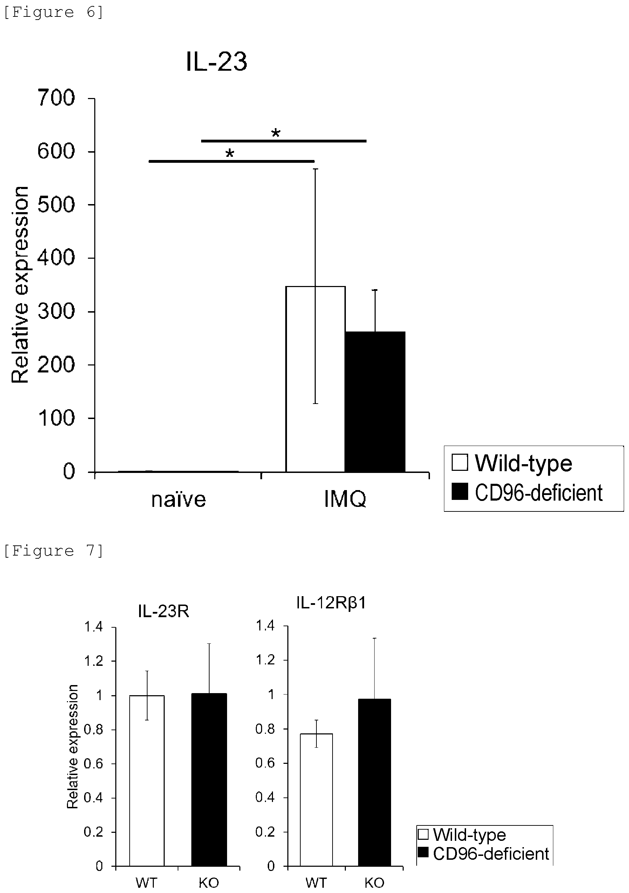

Next, the amount of IL-23 produced in myeloid cells was examined (Figure 6). The amount of IL-23 produced for both the wild-type mice and the CD96-deficient mice was drastically increased in the imiquimod group compared with the naive group, though no significant difference was seen between the wild-type mice and the CD96-deficient mice. This suggests that CD96 is not involved in the promotion of IL-23 production by imiquimod.

Further, the expression levels of the IL-23 receptors IL-23R and IL-12β1 in γδT cells were examined (Figure 7). No significant difference in IL-23R expression level and IL-12β1 expression level was seen between the wild-type mice and the CD96-deficient mice. This suggests that CD96 is not involved in the expression level regulation of the IL-23 receptors in γδT cells.

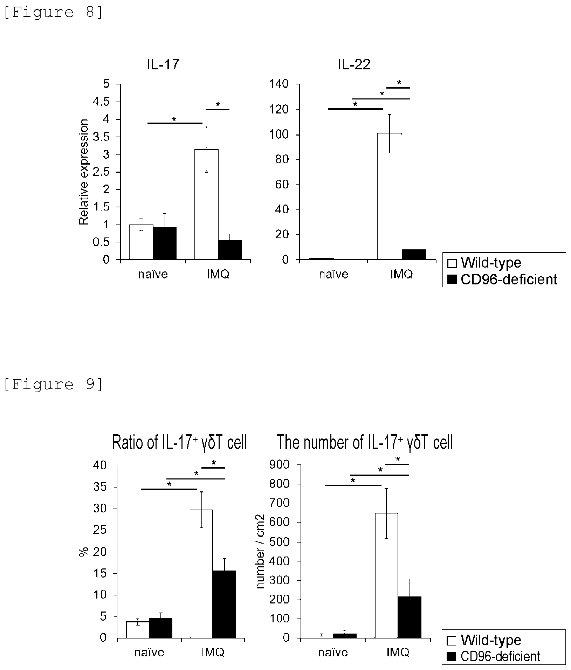

Next, the amounts of IL-17 and IL-22 produced in γδT cells were examined. The amount of IL-17 produced in the wild-type mice was drastically increased in the imiquimod group compared with the naive group, whereas the amount of IL-17 produced in the CD96-deficient mice did not differ between the imiquimod group and the naive group (Figure 8, left). This suggests that CD96 is involved in the promotion of IL-17 in γδT cells by imiquimod. The amount of IL-22 produced for both the wild-type mice and the CD96-deficient mice was increased in the imiquimod group compared with the naive group, though such increase was reduced in the CD96-deficient mice compared with the wild-type mice (Figure 8, right). This suggests that CD96 is involved in the promotion of IL-22 production in γδT cells by imiquimod.

Figure 9 shows the ratio of IL-17-producing cells and the number of IL-17-producing cells in γδT cells. The ratio of IL-17-producing cells in the wild-type mice was increased in the imiquimod group compared with the naive group, whereas such increase in the ratio of IL-17-producing cells by imiquimod was reduced in the CD96-deficient mice. The number of IL-17-producing cells in the wild-type mice was also increased in the imiquimod group compared with the naive group, whereas such increase in the number of IL-17-producing cells by imiquimod was reduced in the CD96-deficient mice. This suggests that CD96 is involved in increase in IL-17-producing cells in γδT cells by imiquimod.

These results indicated that CD96 was largely involved in the pathological conditions of psoriasis in the imiquimod-induced psoriasis models. In the mechanism thereof, CD96 is considered to play an important role in the step of production of IL-17 by γδT cells activated by IL-23, without being involved in the step of production of IL-23 in myeloid cells such as dendritic cells and the step of production of IL-23 receptors in γδT cells, and to increase IL-17 production. Thus, provided that CD96 signals are inhibited, it is expected that IL-17 production can be suppressed and psoriasis can be treated or prevented. Further, provided that signals from CD96 are inhibited, it is expected that a disease or a pathological condition involving the immune response of an IL-17-producing cell, other than psoriasis, can also be treated or prevented.

### <IL-17 production in mouse spleen-derived γδT cell>

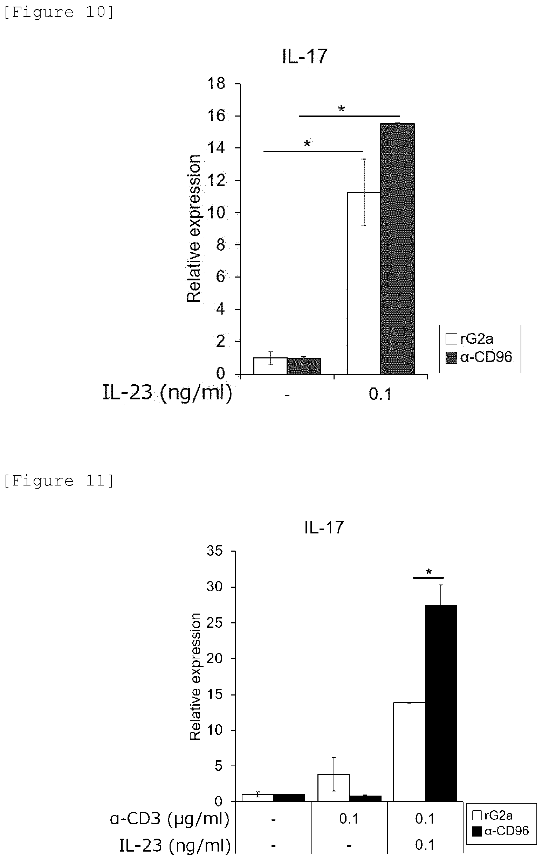

Next, the mechanism under which CD96 increased the IL-17 production of γδT cells was analyzed using wild-type mouse spleen-derived γδT cells. Figure 10 shows a graph in which the amount of IL-17 produced was compared in the presence or absence of addition of IL-23. As is evident, IL-23 increased the amount of IL-17 produced in the γδT cells. Although CD96 was stimulated with an anti-CD96 antibody (a-CD96) immobilized on a plate, no significant difference in the amount of IL-17 produced was seen as compared with the case of a control antibody rat IgG2a (rG2a). This indicated that CD96 does not potentiate IL-23 signals which increase IL-17 production. *In vivo* CD96 recognizes CD155 on target cells for activation, whereas *in vitro* CD96 can be stimulated with an immobilized anti-CD96 antibody.

### <Mechanism of IL-17 production in γδT cell>

It has been reported for imiquimod-induced psoriasis models that TCR signals are necessary for the induction of psoriasis pathological conditions because the psoriasis pathological conditions are reduced by the administration of a T cell receptor (TCR) signal suppressor AX-024 (Science translational medicine, 2016). This suggests that T cell activation mediated by not only cytokines but TCR is essential for the induction of psoriasis pathological conditions.

Accordingly, CD3 that formed a complex with a T cell receptor was stimulated with an immobilized anti-CD3 antibody to activate γδT cells, and the influence of CD96 thereon was examined. *In vivo* T cells are activated through a T cell receptor which recognizes an antigen presented by cells of the natural immune system (particularly, dendritic cells), whereas *in vitro* T cells can be activated by the stimulation of CD3 of a formed complex with a T cell receptor using an immobilized anti-CD3 antibody.

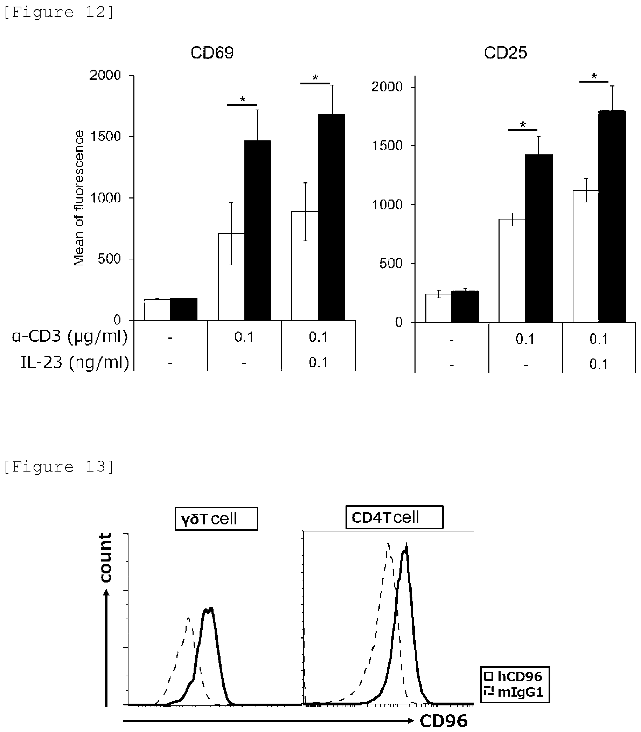

As shown in Figure 11, only CD3 signals did not change the amount of IL-17 produced, while the addition of an immobilized anti-CD3 antibody (a-CD3) as well as IL-23 largely elevated IL-17 production. The IL-17 production was further largely elevated when CD96 was stimulated with an immobilized anti-CD96 antibody (a-CD96). In this respect, Figure 12 shows results of examining whether γδT cells were activated. The stimulation of CD96 by the immobilized anti-CD96 antibody (a-CD96) increased the expression levels of T cell activation markers CD69 and CD25. This suggests that the IL-17 production of γδT cells is increased by activating the γδT cells with CD96 which potentiates the T cell receptor signals of the γδT cells. Thus, provided that CD96 signals are inhibited, it is expected that the activation of γδT cells is suppressed without potentiating the T cell receptor signals of the γδT cells so that the IL-17 production of the γδT cells is suppressed.

### <Expression of CD96 in human PBMC-derived γδT cell and CD4+ T cell>

The left graph of Figure 13 indicated that CD96 is expressed in human PBMC-derived γδT cells. The right graph of Figure 13 indicated that CD96 is also expressed in human PBMC-derived CD4+ T cells.

### <IL-17 production in human PBMC-derived γδT cell>

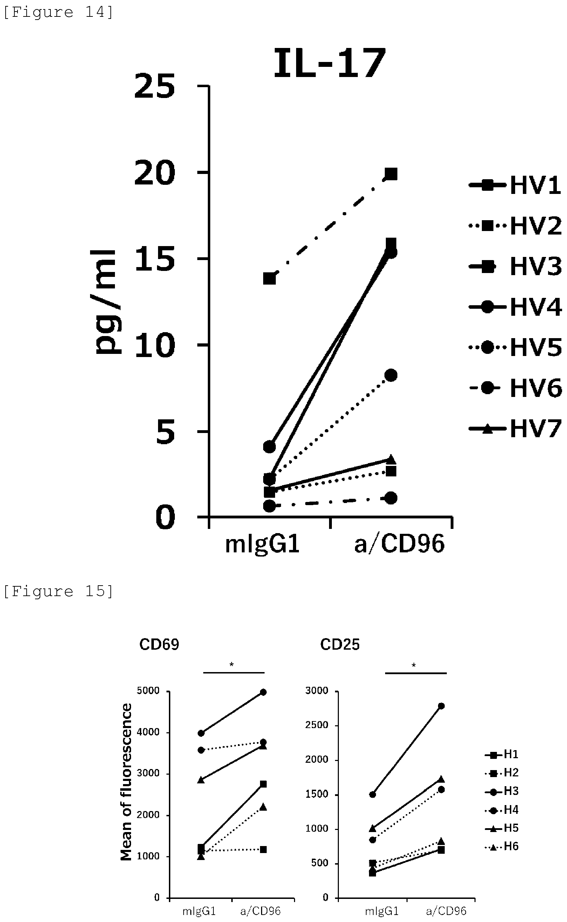

As shown in Figure 14, for γδT cells derived from all seven test subjects (human volunteers; HV1 to HV7), the stimulation of CD96 with an immobilized anti-CD96 antibody (a/CD96) largely elevated IL-17 production as compared with a control (mIgG1). This result suggests that, as in mouse spleen-derived γδT cells, CD96 potentiates the T cell receptor signals of human PBMC-derived γδT cells so that IL-17 production is increased.

### <Activation of human PBMC-derived γδT cells>

As shown in Figure 15, for γδT cells derived from all six test subjects (HV1 to HV6), the stimulation of CD96 with an immobilized anti-CD96 antibody (a/CD96) increased the expression levels of T cell activation markers CD69 and CD25 as compared with a control (mIgG1). This suggests that the IL-17 production of γδT cells is increased through CD96 which potentiates the T cell receptor signals of the γδT cells and thereby activates the γδT cells.

### <IL-17 production in human PBMC-derived CD4+ T cell>

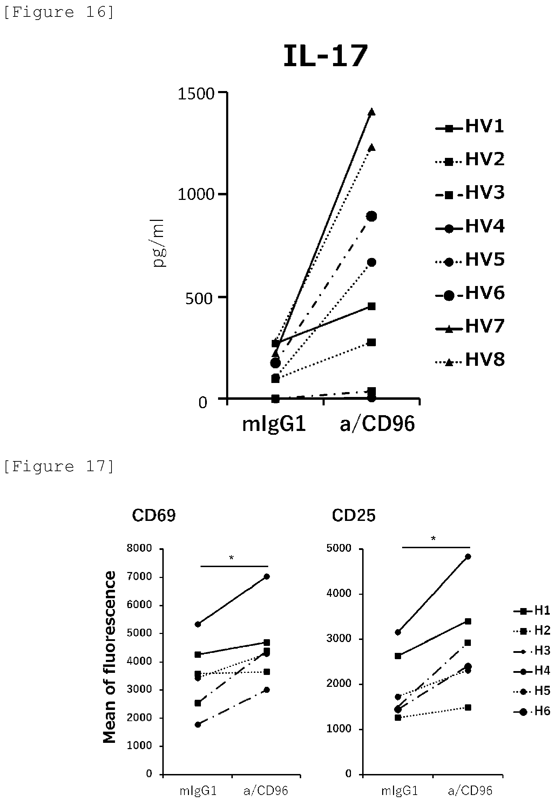

As shown in Figure 16, for CD4+ T cells derived from all eight test subjects (HV1 to HV8), the stimulation of CD96 with an immobilized anti-CD96 antibody (a/CD96) largely elevated IL-17 production as compared with a control (mIgG1). This result suggests that in human PBMC-derived CD4+ T cells as well, CD96 potentiates T cell receptor signals so that IL-17 production is increased.

### <Activation of human PBMC-derived CD4+ T cell>

As shown in Figure 17, for CD4+ T cells derived from all six test subjects (HV1 to HV6), the stimulation of CD96 with an immobilized anti-CD96 antibody (a/CD96) increased the expression levels of T cell activation markers CD69 and CD25 as compared with a control (mIgG1). This suggests that the IL-17 production of human PBMC-derived CD4+ T cells is increased through CD96 which potentiates the T cell receptor signals and thereby activates the CD4+ T cells.

### <Action of anti-CD96 neutralizing antibody on IL-17 production of human PBMC-derived CD4+ T cell>

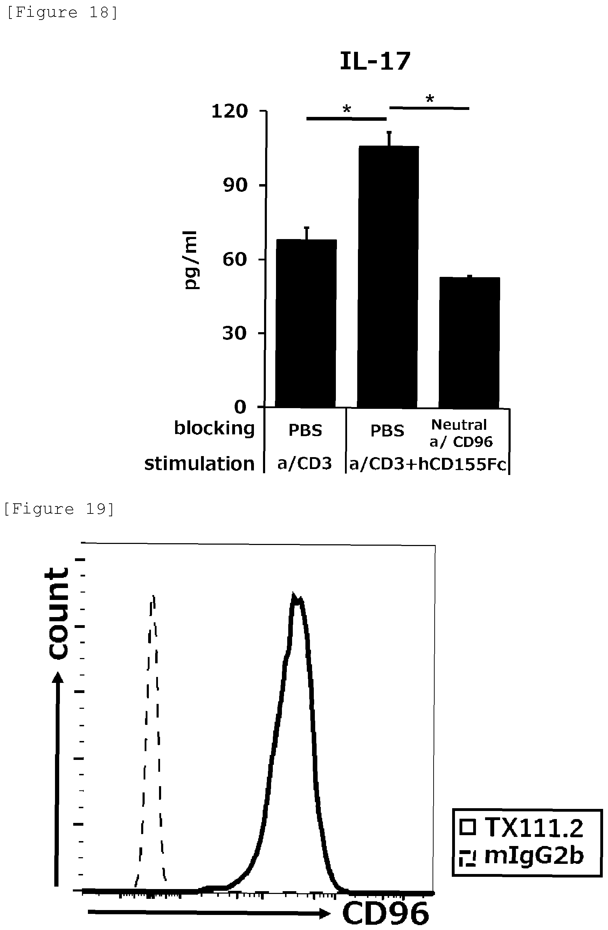

In Figure 18, the row "stimulation" shows a material used in immobilization in order to stimulate CD4+ T cells. The row "blocking" shows a material mixed in advance with CD4+ T cells in order to block the binding of other molecules to CD96. The stimulation of CD96 with an anti-CD3 antibody (a/CD3) as well as hCD155-Fc immobilized on a plate significantly increased IL-17 production in the CD4+ T cells as compared with stimulation with only the immobilized anti-CD3 antibody. The binding of hCD155-Fc to CD96 was blocked when the CD4+ T cells were mixed in advance with an anti-CD96 neutralizing antibody (neutral a/CD96), which significantly suppressed IL-17 production in the CD4+ T cells as compared with the case of being mixed in advance with PBS. Specifically, the anti-CD96 neutralizing antibody suppressed IL-17 production, which is an immune response of human PBMC-derived CD4+ T cells. This result suggested the possibility that an anti-CD96 neutralizing antibody can be used as an immune response suppressant for an IL-17-producing cell and furthermore, is effective for the prevention and treatment of a disease in which inflammation is provoked by IL-17.

### <Function of CD96>

These results indicated that CD96 functions as an immune response activation receptor in IL-17-producing cells such as γδT cells or CD4+ T cells. Further, this function of CD96 in IL-17-producing cells was found common between mice and humans.

Thus, provided that CD96 signals are inhibited both in mice and in humans, it is expected that the activation of γδT cells or CD4+ T cells is suppressed without potentiating the T cell receptor signals of the γδT cells or the CD4+ T cells so that IL-17 production is suppressed.

Much remains unknown about the role of CD96 in an immunological control system. For example, in mouse NK cells, CD96 is known to function as an immunosuppressive receptor. Hence, the possibility has been reported that an anti-CD96 antibody can be used as a drug reducing immunosuppression, i.e., activating immune response. In human NK cells, CD96 was originally reported as a receptor that activated immunity (Anja Fuchs et al., J Immunol, 2004, 172, 3994-3998, Cutting Edge: CD96 (Tactile) Promotes NK Cell-Target Cell Adhesion by Interacting with the Poliovirus Receptor (CD155)). However, now, whether CD96 in human NK cells functions as an activation receptor or a suppressive receptor is under discussion. Hence, in the case of targeting K cells, an anti-CD96 antibody might not activate immune response in humans. On the other hand, the present inventors found that CD96 in IL-17-producing cells functions as an immune response activation receptor both in mice and in humans. Specifically, since the function of CD96 in IL-17-producing cells is considered to be common across species, the immune response suppressant for an IL-17-producing cell of the present invention is expected to similarly suppress immune response both in mice and in humans.

### <Anti-CD96 neutralizing antibody TX111.2>

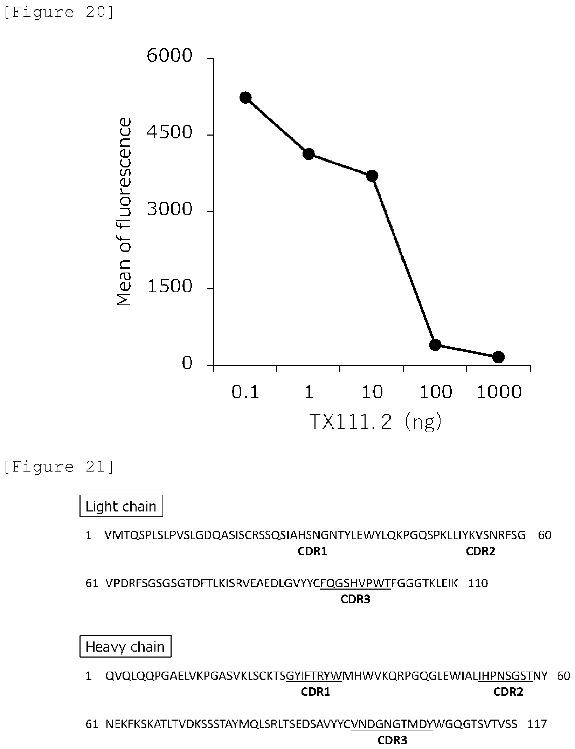

Figure 19 indicated that the anti-CD96 antibody TX111.2 has specificity for CD96. In Figure 20, a fluorescence value was decreased in a manner dependent on the concentration of the anti-CD96 antibody TX111.2, indicating that the anti-CD96 antibody TX111.2 competes with a CD96 natural ligand CD155 for binding to CD96. This indicated that the anti-CD96 antibody TX111.2 is an anti-CD96 neutralizing antibody which inhibits the binding of CD96 to CD155.

Figure 21 shows the amino acid sequences of the heavy chain variable region and light chain variable region of TX111.2. In Figure 21, CDR (complementarity determining region) 1 to CDR3 in the heavy chain variable region and the light chain variable region were underlined.

### <Prophylactic administration experiment of anti-mouse CD96 neutralizing antibody to imiquimod-induced psoriasis mouse model>

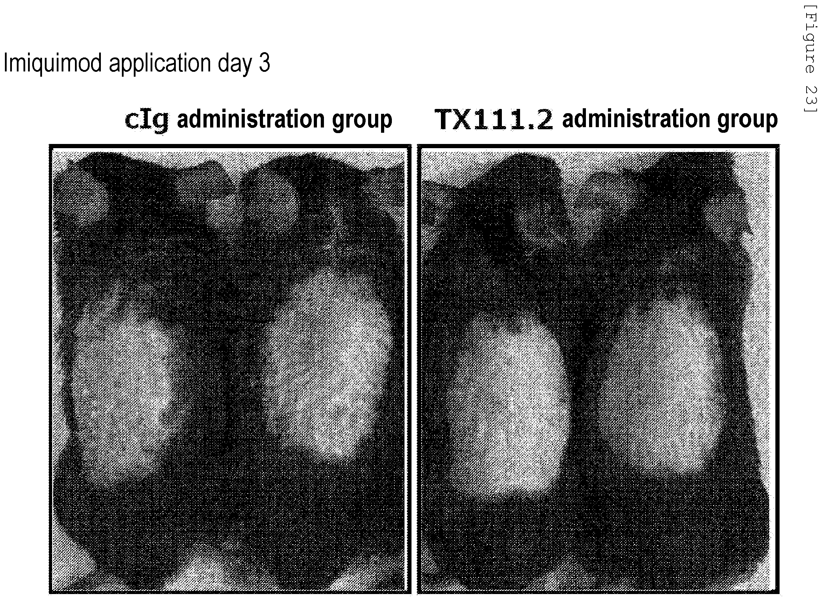

Figure 23 shows photographs of the backs on day 3 from the start of application of a TX111.2 administration group and a cIg administration group of imiquimod-induced psoriasis models. In the cIg administration group, the skin became red with scaling confirmed. In the TX111.2 administration group, the skin became red with scaling confirmed, though the degrees thereof were reduced as compared with the cIg administration group.

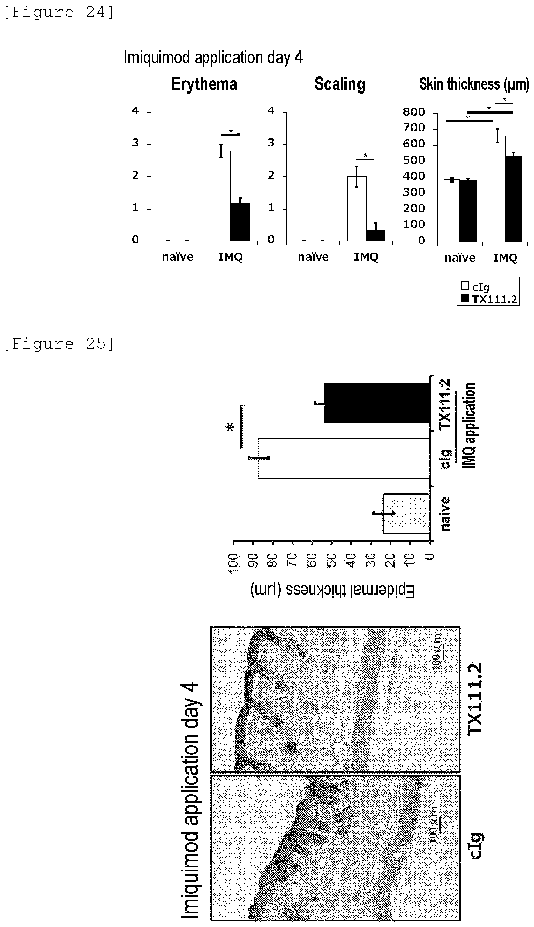

Figure 24 shows graphs in which erythema, scaling, and a skin thickness on day 4 from the start of application were compared between the TX111.2 administration group and the cIg administration group. The ordinates of the graphs about erythema and scaling depict an erythema score and a scaling score, respectively, and the ordinate of the graph about a skin thickness depicts a skin thickness (µm). Neither erythema nor scaling was seen in both the TX111.2 administration group and the cIg administration group in the naive group.

The erythema scores in the imiquimod group (IMQ) were higher for both the TX111.2 administration group and the cIg administration group than those in the naive group, whereas the erythema score of the TX111.2 administration group was significantly lower than that of the cIg administration group.

The scaling scores in the imiquimod group were higher for both the TX111.2 administration group and the cIg administration group than those in the naive group, whereas the scaling score of the TX111.2 administration group was significantly lower than that of the cIg administration group.

The skin in the imiquimod group was thicker for both the TX111.2 administration group and the cIg administration group than that in the naive group, whereas the skin of the TX111.2 administration group was significantly thinner than that of the cIg administration group.

These results indicated that the pathological conditions of erythema, scaling, and skin hyperplasia induced by imiquimod were reduced by TX111.2 administration.

Figure 25 (left) shows photographs of HE-stained tissue preparations of the mouse back skin on day 4 from the start of imiquimod application. Pathological conditions characteristic of psoriasis, such as epidermal hyperplasia, parakeratosis, and the infiltration of neutrophils and mononuclear cells into the dermis, were observed in the cIg administration group, whereas these pathological conditions were reduced in the TX111.2 administration group. An epidermal thickness was graphed in Figure 25 (right). The epidermal thickness of the cIg administration group was approximately 4 times in the imiquimod group compared with the naive group, whereas the epidermal thickness of the TX111.2 administration group was approximately 2 times therein. Thus, epidermal hyperplasia caused by imiquimod was reduced by TX111.2 administration.

These results indicated that the anti-mouse CD96 neutralizing antibody TX111.2 has a prophylactic effect on psoriasis.

### <Therapeutic administration experiment of anti-mouse CD96 neutralizing antibody to imiquimod-induced psoriasis mouse model >

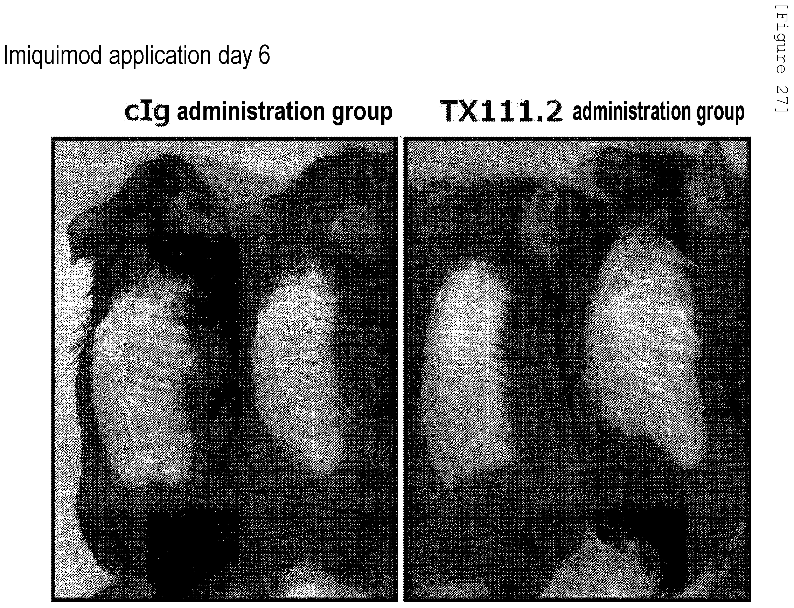

Figure 27 shows photographs of the backs on day 6 from the start of application of a TX111.2 administration group and a cIg administration group of imiquimod-induced psoriasis models. In the cIg administration group, the skin became red with scaling confirmed. In the TX111.2 administration group, the skin became red with scaling confirmed, though the degrees thereof were reduced as compared with the cIg administration group.

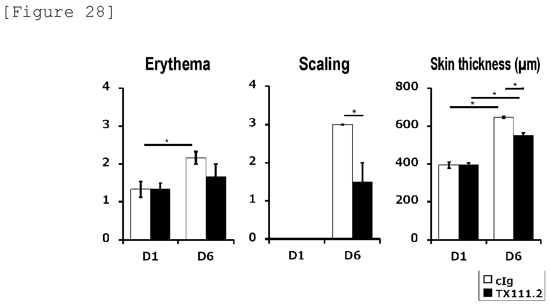

Figure 28 shows graphs in which erythema, scaling, and a skin thickness on days (D1) and 6 (D6) from the start of application were compared between the TX111.2 administration group and the cIg administration group. The ordinates of the graphs about erythema and scaling depict an erythema score and a scaling score, respectively, and the ordinate of the graph about a skin thickness depicts a skin thickness (µm).

The erythema scores on day 6 (D6) from the start of application were higher for both the TX111.2 administration group and the cIg administration group than those on day 1 (D1) from the start of application, whereas the erythema score of the TX111.2 administration group was lower than that of the cIg administration group.

The scaling scores which were zero on day 1 (D1) from the start of application were higher on day 6 (D6) from the start of application for both the TX111.2 administration group and the cIg administration group, whereas the scaling score of the TX111.2 administration group was significantly lower than that of the cIg administration group.

The skin on day 6 (D6) from the start of application was thicker for both the TX111.2 administration group and the cIg administration group than that on day 1 (D1) from the start of application, whereas the skin of the TX111.2 administration group was significantly thinner than that of the cIg administration group.

These results indicated that the pathological conditions of erythema, scaling, and skin hyperplasia induced by imiquimod were reduced by TX111.2 administration.

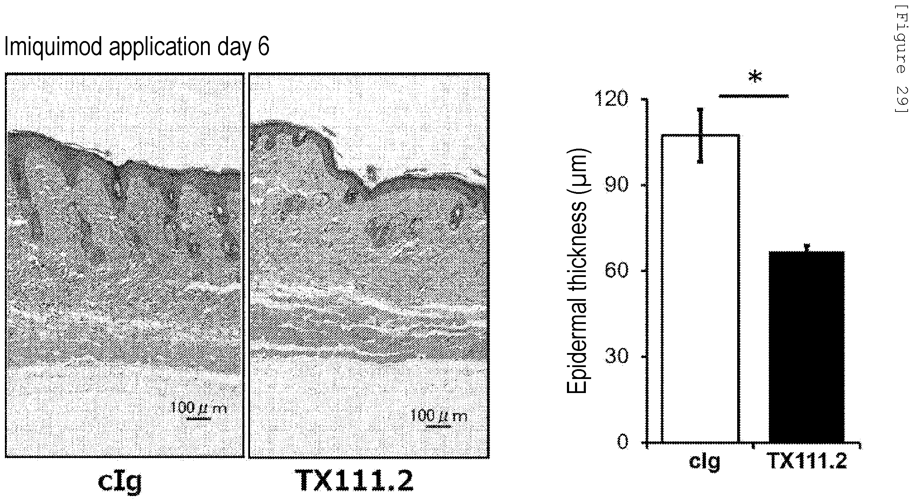

Figure 29 (left) shows photographs of HE-stained tissue preparations of the mouse back skin on day 6 from the start of imiquimod application. Pathological conditions characteristic of psoriasis, such as epidermal hyperplasia, parakeratosis, and the infiltration of neutrophils and mononuclear cells into the dermis, were observed in the cIg administration group, whereas these pathological conditions were reduced in the TX111.2 administration group. An epidermal thickness was graphed in Figure 29 (right). Epidermal hyperplasia was significantly reduced in the TX111.2 administration group compared with the cIg administration group.

These results indicated that the anti-mouse CD96 neutralizing antibody TX111.2 has a therapeutic effect on psoriasis.

### <Administration experiment of anti-mouse CD96 neutralizing antibody to EAE mouse model>

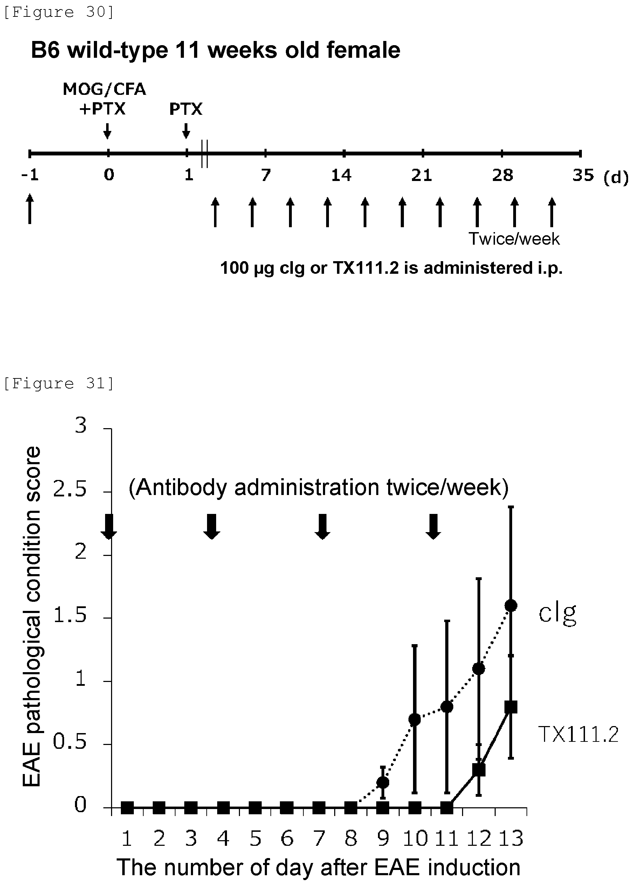

Figure 31 shows change in EAE pathological condition score in a TX111.2 administration group and a cIg administration group of EAE mouse models. In the cIg administration group, the EAE pathological condition score was elevated from day 9 after EAE induction. In the TX111.2 administration group, the elevation of the EAE pathological condition score started on day 12 after EAE induction. The EAE pathological condition score of the TX111.2 administration group was always lower than that of the cIg administration group during the test period.

This result indicated that the anti-mouse CD96 neutralizing antibody TX111.2 has prophylactic and therapeutic effects on multiple sclerosis.

The present inventors newly found that CD96 is expressed in γδT cells producing IL-17. They also found that in γδT cells and CD4+ T cells, CD96 functions as an activation receptor and further, CD96 signals promote IL-17 production. Furthermore, the anti-CD96 neutralizing antibody was found, using mouse models, effective for the prevention and treatment of psoriasis and multiple sclerosis, which are diseases in which inflammation is provoked by IL-17.

Existing IL-17 inhibitors such as anti-IL-17A antibodies or anti-IL-17A receptor antibodies suppress the systemic immune activity of IL-17 and might therefore produce adverse reactions such as infection or neutropenia. The present inventors revealed the mechanism under which IL-17 is produced in particular cells at an inflammatory site, and found an immunosuppressant which suppresses the production of IL-17 by inhibiting a portion of the mechanism. The immunosuppressant of the present invention acts specifically for an inflammatory site and is therefore expected to have fewer adverse reactions.

### Industrial Applicability

The present invention can provide an immune response suppressant for suppressing the immune response of IL-17-producing cells which causes a pathological condition such as psoriasis, and a medicament for treating or preventing a disease or a pathological condition involving the immune response of an IL-17-producing cell. Also, a method for inducing immune response in γδT cells can be provided. Further, a method for evaluating a medicament for treating or preventing a disease or a pathological condition involving the immune response of an IL-17-producing cell can be provided. A method for producing IL-17 by inducing immune response in γδT cells can be provided.

## Claims

1. An immune response suppressant for an IL-17-producing cell, comprising a substance that inhibits the binding of CD96 to at least one protein selected from CD155 and CD111.

2. The immune response suppressant for an IL-17-producing cell according to claim 1, wherein the substance that inhibits the binding of CD96 to at least one protein selected from CD155 and CD111 is an anti-CD96 antibody.

3. The immune response suppressant for an IL-17-producing cell according to claim 1 or 2, wherein the IL-17-producing cell is a γδT cell.

4. The immune response suppressant for an IL-17-producing cell according to any one of claims 1 to 3, wherein the immune response suppressant for an IL-17-producing cell is an IL-17 production suppressant or an IL-22 production suppressant.

5. A medicament for treating or preventing a disease or a pathological condition involving the immune response of an IL-17-producing cell, comprising an immune response suppressant for an IL-17-producing cell according to any one of claims 1 to 4.

6. The medicament according to claim 5, wherein the disease or the pathological condition involving the immune response of an IL-17-producing cell is psoriasis, rheumatoid arthritis, atopic dermatitis, multiple sclerosis, cerebral infarction, nephritis, lung injury, pulmonary fibrosis or skin tumor.

7. A method comprising a step (A) of culturing at least one of a γδT cell and a CD4-positive T cell together with IL-23, an anti-CD3 antibody capable of stimulating a TCR/CD3 complex and an anti-CD96 antibody capable of stimulating CD96.

8. The method according to claim 7, further comprising a step (B) of evaluating IL-17 after performing the step (A).

9. A method for evaluating a medicament for treating or preventing a disease or a pathological condition involving the immune response of an IL-17-producing cell, comprising a method according to claim 8.

10. The method for evaluating a medicament according to claim 9, wherein the disease or the pathological condition involving the immune response of an IL-17-producing cell is psoriasis, rheumatoid arthritis, atopic dermatitis, multiple sclerosis, cerebral infarction, nephritis, lung injury, pulmonary fibrosis or skin tumor.

11. A method for producing IL-17, comprising a step (A) of culturing at least one of a γδT cell and a CD4-positive T cell together with IL-23, an anti-CD3 antibody capable of stimulating a TCR/CD3 complex and an anti-CD96 antibody capable of stimulating CD96.
